(19) Europäisches Patentamt
European Patent Office
Office européen des brevets

(11) EP 4 578 866 A1

(12) EUROPEAN PATENT APPLICATION
published in accordance with Art. 153(4) EPC

(43) Date of publication:
**02.07.2025 Bulletin 2025/27**

(21) Application number: **23857360.4**

(22) Date of filing: **23.08.2023**

(51) International Patent Classification (IPC):
***C07K 2/00*** *(2006.01)*

(52) Cooperative Patent Classification (CPC):
**C07K 2/00**

(86) International application number:
**PCT/JP2023/030224**

(87) International publication number:
**WO 2024/043251 (29.02.2024 Gazette 2024/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.08.2022 JP 2022132148**

(71) Applicant: **FUJIFILM Corporation**
**Tokyo 106-8620 (JP)**

(72) Inventors:
- **TAMURA Takashi**
  **Ashigarakami-gun, Kanagawa 258-8577 (JP)**
- **KANEKO Mai**
  **Ashigarakami-gun, Kanagawa 258-8577 (JP)**
- **YOSHIMITSU Yuji**
  **Ashigarakami-gun, Kanagawa 258-8577 (JP)**
- **TSUMURA Kyosuke**
  **Ashigarakami-gun, Kanagawa 258-8577 (JP)**
- **SUZUKI Koo**
  **Ashigarakami-gun, Kanagawa 258-8577 (JP)**
- **OHASHI Noriyuki**
  **Ashigarakami-gun, Kanagawa 258-8577 (JP)**
- **HASHIMOTO Ichihiko**
  **Ashigarakami-gun, Kanagawa 258-8577 (JP)**
- **MIYAHARA Kenta**
  **Ashigarakami-gun, Kanagawa 258-8577 (JP)**
- **KOCHI Masahiro**
  **Ashigarakami-gun, Kanagawa 258-8577 (JP)**
- **HORIGOME Hiroki**
  **Ashigarakami-gun, Kanagawa 258-8577 (JP)**

(74) Representative: **Klunker IP Patentanwälte PartG mbB**
**Destouchesstraße 68**
**80796 München (DE)**

# (54) METHOD FOR PREDICTING CELL MEMBRANE PERMEABILITY OF CYCLIC PEPTIDE

(57) An object of the present invention is to provide a method for predicting cell membrane permeability of a cyclic peptide, which enables versatile design of a cyclic peptide with cell membrane permeability. According to the present invention, there is provided a method for predicting cell membrane permeability of a cyclic peptide, the method including a first step of acquiring a structure of the cyclic peptide; a second step of calculating a molecular shape factor r which is calculated by Expression (1) after a step of carrying out an ellipsoidal approximation for obtaining each of axis lengths a, b, and c in a case where an axis length in a longest axis direction of a main chain structure is denoted by a, and axis lengths in two other directions which are orthogonal to a and are orthogonal to each other are denoted by b and c in the structure acquired in the first step; and a third step of determining that the cyclic peptide having the molecular shape factor r in a range of 0.4 to 0.6 has cell membrane permeability.

$$r = \frac{2\sqrt{b^2 + c^2}}{\sqrt{a^2 + b^2} + \sqrt{c^2 + a^2}} \qquad (1)$$

EP 4 578 866 A1

## Description

Technical Field

The present invention relates to a method for predicting cell membrane permeability of a cyclic peptide based on a structure of the cyclic peptide.

Background Art

In recent years, a cyclic peptide has been attracting attention primarily in the field of pharmaceuticals due to having resistance to metabolic enzymes and specific protein binding properties. In addition, cyclosporin A (molecular weight: 1202.6), which is a cyclic peptide composed of 11 amino acid residues, permeates the intestinal membranes and cell membranes, so it is expected that a cyclic peptide having a molecular weight of about 1000 will be able to permeate the cell membranes. This indicates that, in a case where a cyclic peptide can compensate for the low cell membrane permeability of an antibody pharmaceutical, which is known to be highly effective as a biopharmaceutical, while exhibiting the same level of specific protein binding properties as those of the antibody pharmaceutical, there is a possibility that the cyclic peptide can be used to create a novel pharmaceutical. Unfortunately, it is difficult to design a cell membrane permeable cyclic peptide having a molecular weight of more than 1000, and there are few examples in which the cell membrane permeable cyclic peptide has been used for industrial purposes such as pharmaceuticals.

Cyclization of a peptide has long been known as a method for increasing the cell membrane permeability. For example, it has been suggested that a cyclic peptide, which has a ring structure introduced into a main chain thereof, has increased cell membrane permeability in part because the polarity of an amide group is offset by the formation of intramolecular hydrogen bonds (Non-Patent Document 1). In addition, the improvement of cell membrane permeability has also been demonstrated for a cyclic peptide having a staple structure (Non-Patent Document 2). Furthermore, it has been proposed to enhance the cell membrane permeability by controlling a side chain structure of a cyclic peptide (Patent Documents 1,2 and 3) or a substituent structure on an amide group (Non-Patent Document 3). Furthermore, a study has been reported that showed a correlation between the polarity of a cyclic peptide as a parameter and the cell membrane permeability (Non-Patent Document 4). On the other hand, it has been suggested that cyclosporin A changes its structure to different ones in water and in the cell membrane, and adopts a structure in the cell membrane that is advantageous for the cell membrane permeability, thereby increasing the cell membrane permeability (Non-Patent Document 5).

Prior Art Documents

Patent Documents

Patent Document 1: WO2018/225864A
Patent Document 2: WO2020/122182A
Patent Document 3: WO2015/030014A


Non-Patent Documents

Non-Patent Document 1: Nat. Chem. 2016, 8, 1105-1111
Non-Patent Document 2: Proc. Natl. Acad. Sci. USA 2013, 110, E3445
Non-Patent Document 3: Acc. Chem. Res. 2008, 41, 1331-1342, Nat. Chemical Biology 2011, 7, 810-817
Non-Patent Document 4: ACS Med. Chem. Lett. 2014, 5, 1167-1172, J. Med. Chem. 2018, 61, 4189-4202, J. Med. Chem. 2018, 61, 11169-11182
Non-Patent Document 5: J. Am. Chem. Soc. 2006, 128, 14073-14080, J. Chem. Inf. Model. 2016, 56, 1547-1562, J. Phys. Chem. B 2018, 122, 2261-2276


## SUMMARY OF THE INVENTION

### Object to be solved by the invention

The above-mentioned related art methods are not necessarily effective as guidelines for designing a cyclic

peptide with cell membrane permeability, and further design techniques for cell membrane permeation are required. In addition, significant costs are being spent on exploratory research, and it is desired to predict the characteristics of a cyclic peptide in advance without preparing the cyclic peptide. An object of the present invention is to provide a method for predicting cell membrane permeability of a cyclic peptide, which enables versatile design of a cyclic peptide with cell membrane permeability.

Means for solving the object

As a result of extensive studies to achieve the foregoing object, the present inventors have found that, in a case where a molecular shape factor r for a structure of a cyclic peptide, which is calculated by Expression (1) defined in the present specification, is in a range of 0.4 to 0.6, the cyclic peptide has high cell membrane permeability. The present invention has been completed based on the above findings. According to the present invention, the following inventions are provided.

<1> A method for predicting cell membrane permeability of a cyclic peptide, the method comprising a first step of acquiring a structure of the cyclic peptide; a second step of calculating a molecular shape factor r which is calculated by Expression (1) after a step of carrying out an ellipsoidal approximation for obtaining each of axis lengths a, b, and c in a case where an axis length in a longest axis direction of a main chain structure is denoted by a, and axis lengths in two other directions which are orthogonal to a and are orthogonal to each other are denoted by b and c in the structure acquired in the first step; and

$$r = \frac{2\sqrt{b^2 + c^2}}{\sqrt{a^2 + b^2} + \sqrt{c^2 + a^2}} \qquad (1)$$

a third step of determining that the cyclic peptide having the molecular shape factor r in a range of 0.4 to 0.6 has cell membrane permeability.

<2> The method according to <1>, in which, in the first step, the structure of the cyclic peptide is acquired by X-ray crystallography.

<3> The method according to <1>, in which, in the first step, the structure of the cyclic peptide is acquired by molecular dynamics calculation.

<4> The method according to <1>, in which, in the first step, the structure of the cyclic peptide is acquired by acquiring positional structural information of the cyclic peptide by two-dimensional $^1$H-NMR measurement and then carrying out structuring by computational chemistry based on the acquired positional structural information.

<5> The method according to <4>, in which the two-dimensional $^1$H-NMR measurement is a measurement by at least one of nuclear Overhauser effect spectroscopy, also referred to as NOESY, or rotating frame nuclear Overhauser effect spectroscopy, also referred to as ROESY.

<6> The method according to <4>, in which the two-dimensional $^1$H-NMR measurement is carried out at a temperature of 20°C to 60°C.

<7> The method according to <4>, in which the two-dimensional $^1$H-NMR measurement is carried out in dimethyl sulfoxide, dimethylformamide, dimethylacetamide, dichloromethane, chloroform, water, methanol, ethanol, propanol, tetrahydrofuran, or acetonitrile.

<8> The method according to <4>, in which the computational chemistry is a molecular dynamics method.

<9> The method according to any one of <1> to <8>, in which the cyclic peptide is non-ionic in a physiological environment.

<10> The method according to any one of <1> to <8>, in which the main chain structure of the cyclic peptide contains a sulfur atom.

Effect of the invention

According to the present invention, a cyclic peptide compound having cell membrane permeability can be obtained.

## BRIEF DESCRIPTION OF THE DRAWINGS

Fig. 1 shows initial structures of compound 1, compound 2, and cyclosporin A.
Fig. 2 shows final structures of compound 1, compound 2, and cyclosporin A.
Fig. 3 shows final structures of compound 1, compound 2, and cyclosporin A.
Fig. 4 shows an ellipsoid for compound 1.
Fig. 5 shows an ellipsoid for compound 2.
Fig. 6 shows an ellipsoid for cyclosporin A.
Fig. 7 shows three-dimensionally structured cyclosporin A and isocyclosporin.
Fig. 8 shows structures of cyclosporin A and isocyclosporin after structure optimization by MD calculation using three-dimensional structures as initial structures.
Fig. 9 shows structures of most stabilized cyclosporin A and isocyclosporin.
Fig. 10 shows a main chain structure by MD calculation and a main chain structure by NMR + MD calculation.
Fig. 11 shows an ellipsoid for cyclosporin A.
Fig. 12 shows an ellipsoid for isocyclosporin.

## EMBODIMENTS FOR CARRYING OUT THE INVENTION

Hereinafter, the present invention will be described in more detail.
In the present specification, "to" shows a range including numerical values described before and after "to" as a minimum value and a maximum value, respectively.
The method for predicting cell membrane permeability of a cyclic peptide according to the embodiment of the present invention includes

a first step of acquiring a structure of a cyclic peptide;
a second step of calculating a molecular shape factor r which is calculated by Expression (1) after a step of carrying out an ellipsoidal approximation for obtaining each of axis lengths a, b, and c in a case where an axis length in a longest axis direction of a main chain structure is denoted by a, and axis lengths in two other directions which are orthogonal to a and are orthogonal to each other are denoted by b and c in the structure acquired in the first step; and

$$r = \frac{2\sqrt{b^2 + c^2}}{\sqrt{a^2 + b^2} + \sqrt{c^2 + a^2}} \qquad (1)$$

a third step of determining that the cyclic peptide having the molecular shape factor r in a range of 0.4 to 0.6 has cell membrane permeability.

According to the prediction method according to the embodiment of the present invention, it is possible to grasp in advance a cyclic peptide having high intracellular permeability before synthesis, and it is possible to design a cell membrane-permeable peptide, which has been difficult to do in the related art. The cyclic peptide compound obtained by the prediction method according to the embodiment of the present invention can be used as molecular design knowledge for pharmaceuticals, bioimaging, and culture medium components for cell culture. In addition, according to the present invention, it is possible to reduce research costs.

<First step>

The first step is a step of acquiring the structure of the cyclic peptide.
In the first step, for example,

(1) acquisition of the structure of the cyclic peptide by two-dimensional $^1$H-NMR measurement and computational chemistry,
(2) acquisition of the structure of the cyclic peptide by molecular dynamics calculation, or
(3) acquisition of the structure of the cyclic peptide by X-ray crystallography

can be carried out, but there is no particular limitation as long as the method is capable of acquiring the structure of the cyclic peptide.

(Acquisition of structure of cyclic peptide by two-dimensional $^1$H-NMR measurement and computational chemistry)

In the first step, the structure of the cyclic peptide can be acquired by acquiring positional structural information of the cyclic peptide by two-dimensional $^1$H-NMR measurement and then carrying out structuring by computational chemistry based on the acquired positional structural information.
The two-dimensional $^1$H-NMR measurement is preferably a measurement by at least one of NOESY (nuclear Overhauser effect spectroscopy) or ROESY (rotating frame nuclear Overhauser effect spectroscopy).
Variable temperature NMR, J-coupling, or the like can also be used. The J-coupling is an interaction of a target proton in NMR with a non-equivalent proton on the same carbon or an adjacent carbon. As a result, signals of the target proton appear split. In addition, the correlation between a coupling constant and a dihedral angle is expressed by the Karplus equation, and in a case where the coupling constant is known, the dihedral angle can be obtained.
The two-dimensional $^1$H-NMR measurement is preferably carried out at a temperature of -40°C to 80°C, more preferably carried out at a temperature of 0°C to 80°C, and still more preferably carried out at a temperature of 20°C to 60°C.
The solvent used in the two-dimensional $^1$H-NMR measurement is not particularly limited, and the two-dimensional $^1$H-NMR measurement is preferably carried out in dimethyl sulfoxide, dimethylformamide, dimethylacetamide, dichloromethane, chloroform, water, methanol, ethanol, propanol, tetrahydrofuran, acetonitrile, or a mixture thereof, and more preferably carried out in dimethyl sulfoxide, chloroform, water, or a mixture thereof.
It is preferable that the computational chemistry is a molecular dynamics method. Examples of the molecular dynamics method include, but are not particularly limited to, a classical molecular dynamics (MD) method, a replica exchange MD method, and a first-principles MD method. The molecular dynamics method is a technique for calculating a dynamic behavior of a system consisting of a large number of atoms in contact with a heat bath at a certain temperature by numerically solving the Newton equation based on an interaction between atoms. The molecular dynamics method is divided into a classical MD method and a first-principles MD method, depending on how the interaction between atoms is given. In a case where the interaction between atoms is given by known functions including parameters such as a charge of each atom, a Van der Waals parameter, and a bond length of a covalent bond, the molecular dynamics method is called a classical MD method. In a case where the interaction between atoms is calculated by a molecular orbital method, which explicitly treats electrons, the molecular dynamics method is called a first-principles MD method. There is usually a single heat bath for controlling the temperature of the system used in the classical MD method and the first-principles MD method. However, it is possible to introduce a plurality of heat baths having different temperatures and use the heat baths to accelerate the dynamic behavior of the system, which is called a replica exchange MD method.

(Acquisition of structure of cyclic peptide by molecular dynamics calculation)

In the first step, the structure of the cyclic peptide can be acquired by molecular dynamics calculation.
The method for structuring the initial structure by molecular dynamics calculation (creating a 3D molecular model from a 2D structural formula) can be carried out using software Chem3D, software Open Babel, or the like.

(Acquisition of structure of cyclic peptide by X-ray crystallography)

In the first step, the structure of the cyclic peptide can be acquired by X-ray crystallography. The cyclic peptide is made into a solution using an appropriate solvent, and the solution is concentrated and crystallized to obtain crystals, which are then irradiated with X-rays using an X-ray irradiation device. The obtained diffraction pattern can be subjected to structure optimization/refinement using computational chemistry to acquire the structure of the cyclic peptide.

<Second step>

The second step is a step of calculating a molecular shape factor r which is calculated by Expression (1) after a step of carrying out an ellipsoidal approximation for obtaining each of axis lengths a, b, and c in a case where an axis length in a longest axis direction of a main chain structure is denoted by a, and axis lengths in two other directions which are orthogonal to a and are orthogonal to each other are denoted by b and c in the structure acquired in the first step.

$$r = \frac{2\sqrt{b^2 + c^2}}{\sqrt{a^2 + b^2} + \sqrt{c^2 + a^2}} \qquad (1)$$

<First embodiment>

In a case where the structure of the cyclic peptide is acquired by two-dimensional $^1$H-NMR measurement and computational chemistry in the first step, the first step and the second step can be carried out, for example, as follows.

First, a target cyclic peptide was dissolved in DMSO-d6 to prepare a solution having a concentration of 5 mg/mL. A sample tube used was a SIGEMI tube (BMS-005B), and a sample volume was set to 400 $\mu$L. For 2D-NMR measurement (600 MHz Cryo system, manufactured by Bruker Corporation), the following three types of measurements were carried out for structure assignment: COSY (cosygpppgf, 128 integrations), TOCSY (melvphpp, 128 integrations, expansion time of 80 msec), and NOESY (noesygpphpp, 64 integrations, expansion time of 150 msec, 300 msec). The variable temperature $^1$H-NMR measurement (zg, a total of 64 times) was carried out at each of 25°C, 30°C, 35°C, 40°C, 45°C, and 50°C, and a $\Delta\delta$NH/T (ppb/K) value was calculated from a change in chemical shift value depending on the temperature.

Next, the structure of the cyclic peptide was determined by restraining the structure generated by the molecular dynamics (MD) method using NMR data.

The calculation of the MD method can be carried out using, for example, AmberTools 16. A GAFF force field can be used for van der Waals interactions, and RESP charges calculated by Gaussian 09 can be used for charges. The NMR data (appropriately selected from the main chain dihedral angle and the HH distance) can be used as the restraint condition using the NMR restraint option implemented in AmberTools 16. Calculation of the structure of the cyclic peptide can be carried out according to the following procedure.

(1) 1,000 initial structures having different conformations are prepared for a linear peptide before cyclization of a target cyclic peptide.
(2) Each linear initial structure is cyclized, and then the restraint based on the NMR data is applied at each step. The order is (i) cyclization/short-range HH distance, (ii) medium-range HH distance, and (iii) long-range HH distance, each of which is calculated over 0.2 ns. With this restraint, the 1,000 structures of the cyclic peptide are deformed to match the NMR data as closely as possible within a range in which each structure can occur as a molecule.
(3) Among the 1,000 structures obtained, the structures are assigned priorities in order of satisfying the NMR data. The top 10 are drawn to determine the final structures.

In the structure having the highest priority, the three-dimensional coordinates of atoms belonging to the main chain of the cyclic peptide are represented by ($X_{a,1}$, $X_{a,2}$, $X_{a,3}$).

Here, a is a label that identifies the atoms belonging to the main chain, and takes an integer from 1 to N. N is the total number of atoms belonging to the main chain of the cyclic peptide.

The r value is calculated for the three-dimensional coordinates. The r value can be calculated according to the following procedure.

(1) Using three-dimensional coordinates as an input, the inertia tensor (a 3 $\times$ 3 matrix) is calculated according to the following expression.

$$\begin{pmatrix} I_{11} & I_{12} & I_{31} \\ I_{21} & I_{22} & I_{32} \\ I_{31} & I_{32} & I_{33} \end{pmatrix} = \begin{pmatrix} \sum_{a=1}^{N}\sum_{k=1}^{3}(X_{a,k})^2 - \sum_{a=1}^{N} X_{a,1}X_{a,1} & -\sum_{a=1}^{N} X_{a,2}X_{a,1} & -\sum_{a=1}^{N} X_{a,3}X_{a,1} \\ -\sum_{a=1}^{N} X_{a,2}X_{a,1} & \sum_{a=1}^{N}\sum_{k=1}^{3}(X_{a,k})^2 - \sum_{a=1}^{N} X_{a,2}X_{a,2} & -\sum_{a=1}^{N} X_{a,3}X_{a,2} \\ -\sum_{a=1}^{N} X_{a,3}X_{a,1} & -\sum_{a=1}^{N} X_{a,2}X_{a,3} & \sum_{a=1}^{N}\sum_{k=1}^{3}(X_{a,k})^2 - \sum_{a=1}^{N} X_{a,3}X_{a,3} \end{pmatrix}$$

(2) Eigenvalues of the inertia tensor are calculated. The obtained three eigenvalues are referred to as principal moments of inertia and are represented by ($I_1$, $I_2$, $I_3$).
(3) Using the principal moments of inertia as an input, each of axis lengths a, b, and c (a > b > c) of an ellipsoid with a uniform distribution is calculated according to the following expression.

$$a = \sqrt{\frac{5}{N}\left[\frac{\sum_{i=1}^{3} I_i}{2} - I_1\right]}$$

$$b = \sqrt{\frac{5}{N}\left[\frac{\sum_{i=1}^{3} I_i}{2} - I_2\right]}$$

$$c = \sqrt{\frac{5}{N}\left[\frac{\sum_{i=1}^{3} I_i}{2} - I_3\right]}$$

(4) Using each of axis lengths of the ellipsoid as an input, the molecular shape factor (r) is calculated according to the following expression.

$$r = \frac{2\sqrt{b^2 + c^2}}{\sqrt{c^2 + a^2} + \sqrt{a^2 + b^2}}$$

<Second embodiment>

In a case where the structure of the cyclic peptide is acquired by molecular dynamics calculation in the first step, the first step and the second step can be carried out, for example, as follows.

First, a two-dimensionally drawn structural formula of the cyclic peptide is input into Chem3D to create a three-dimensional structure. Using the present three-dimensional structure as an initial structure, the structure optimization is carried out using, for example, a quantum chemical calculation method (B3LYP/6-31G*, software: Gaussian) to obtain a locally stable structure. In the locally stable structure, an electrostatic field for generating a cyclic peptide is obtained by a quantum chemical calculation method (B3LYP/6-31G*, software: Gaussian), and a point charge (RESP charge) is assigned to each atom so as to reproduce the electrostatic field. Next, the state of covalent bonds between the atoms is analyzed (Amber), and van der Waals parameters (gaff2) are assigned to each atom. These charges and van der Waals parameters are collectively referred to as a force field.

Next, under the present force field, using the present locally stable structure as an initial structure, a molecular dynamics (MD) simulation is carried out in chloroform (software: Gromacs and plumed). As an efficient method for efficiently exploring a wide conformation space, the MD simulation employs a replica exchange MD method in which temperatures higher than room temperature are also used in addition to room temperature as temperatures at the time of the simulation. The temperatures used are six types (six types of replicas) and are as shown in Table 17 of Examples. The present temperature is applied only to the cyclic peptide and 298 K is always applied to chloroform present around the cyclic peptide. The calculation for 300 ns is carried out using a replica exchange MD method to determine the most stable structure. The method described in the first embodiment is applied to the present most stable structure to obtain the inertia tensor, the principal moments of inertia, a, b, and c, and then the r value.

<Third step>

The third step is a step of determining that the cyclic peptide having the molecular shape factor r in a range of 0.4 to 0.6 has cell membrane permeability.

The molecular shape factor r is preferably 0.4 to 0.55.

In the present invention, the cell membrane permeability may be determined using a polar surface area (including, but not limited to, tPSA, 3D-PSA, and EPSA) or a hydrophobicity index (including, but not limited to, cLogP

and cLogD), in addition to the range of values of the molecular shape factor r.

<Cyclic peptide>

The cyclic peptide of the present invention is preferably a peptide represented by Formula (1).

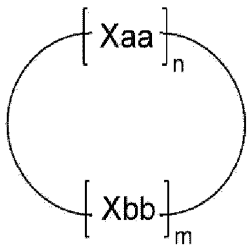

(1)

In the formula, n pieces of Xaa's each independently represent any amino acid residue or any amino acid analog residue,

m pieces of Xbb's each independently represent any amino acid residue or any amino acid analog residue, and
n + m represents an integer of 5 to 50.

n + m represents an integer of 5 to 50, more preferably an integer of 5 to 20, and still more preferably an integer of 9 to 11.

Amino acid refers to a molecule containing both an amino group and a carboxyl group. The amino acid may be any of a natural amino acid or an unnatural amino acid and may be any of D- or L-isomers. The amino acid may be an $\alpha$-amino acid. The $\alpha$-amino acid refers to a molecule containing an amino group and a carboxyl group which are bonded to a carbon designated as an $\alpha$-carbon.

The natural amino acid represents any of alanine (A), arginine (R), asparagine (N), cysteine (C), aspartic acid (D), glutamine (Q), glutamic acid (E), glycine (G), histidine (H), isoleucine (I), leucine (L), lysine (K), methionine (M), phenylalanine (F), proline (P), serine (S), threonine (T), tryptophan (W), tyrosine (Y), or valine (V).

The unnatural amino acid refers to an amino acid other than the above-mentioned 20 types of natural amino acids.

The amino acid analog refers to a molecule that is structurally similar to an amino acid and can be used instead of an amino acid in the production of a cyclic peptide.

Examples of the amino acid analog include, but are not particularly limited to, a $\beta$-amino acid, and an amino acid in which an amino group or a carboxyl group is similarly substituted with a reactive group (for example, a primary amine is substituted with a secondary or tertiary amine, or a carboxyl group is substituted with an ester). The $\beta$-amino acid refers to a molecule containing both an amino group and a carboxyl group in a $\beta$ configuration.

In one example, the amino acid analog is racemic. Either the D-isomer of the amino acid analog may be used, or the L-isomer of the amino acid analog may be used. In addition, the amino acid analog may contain a chiral center in the R or S configuration. Further, the amino group (singular or plural) of the $\beta$-amino acid analog may be substituted with a protective group such as tert-butyloxycarbonyl (BOC group), 9-fluorenylmethyloxycarbonyl (FMOC), or tosyl. Further, the carboxylic acid functional group of the $\beta$-amino acid analog may be protected, for example, as an ester derivative thereof. In addition, a salt of the amino acid analog may be used.

Preferably, the cyclic peptide is non-ionic in a physiological environment. By non-ionic in a physiological environment is meant that the peptide does not have a substituent having a charge in a physiological environment.

Preferably, the main chain structure of the cyclic peptide contains a sulfur atom.

<Method for producing cyclic peptide>

The method for producing a cyclic peptide is not particularly limited. The cyclic peptide may be produced by a method using a cell-free translation system, or may be produced by a chemical synthesis method of a peptide. The chemical synthesis of a peptide can generally be carried out using an automated peptide synthesizer.

The peptide may be synthesized by either a solid phase synthesis method or a liquid phase synthesis method, among which a solid phase synthesis method is preferable. The solid phase synthesis of a peptide is known to those skilled

in the art, and involves, for example, an esterification reaction between a hydroxyl group of a resin having a hydroxyl group and a carboxyl group of a first amino acid (usually a C-terminal amino acid of a desired peptide) in which an $\alpha$-amino group is protected with a protective group. A known dehydration condensation agent such as 1-mesitylenesulfonyl-3-nitro-1,2,4-triazole (MSNT), dicyclohexylcarbodiimide (DCC), or diisopropylcarbodiimide (DIC) can be used as an esterification catalyst. Next, the protective group of the $\alpha$-amino group of the first amino acid is eliminated, a second amino acid in which all functional groups of a main chain except a carboxy group are protected is added, and the carboxy group is activated to bond the first amino acid and the second amino acid. Further, the $\alpha$-amino group of the second amino acid is deprotected, a third amino acid in which all functional groups of a main chain except a carboxy group are protected is added, and the carboxy group is activated to bond the second amino acid and the third amino acid. This process is repeated and in a case where a peptide having a desired length is synthesized, all functional groups are deprotected. Examples of the resin for solid phase synthesis include a Merrifield resin, an MBHA resin, a Cl-Trt resin, a SASRIN resin, a Wang resin, a Rink amide resin, an HMFS resin, an Amino-PEGA resin, and an HMPA-PEGA resin (all manufactured by Merck Sigma-Aldrich Co., LLC). These resins may be washed with a solvent (dimethylformamide (DMF), 2-propanol, methylene chloride, or the like) before use. Examples of the protective group for the $\alpha$-amino group include a benzyloxycarbonyl (Cbz or Z) group, a tert-butoxycarbonyl (Boc) group, a fluorenylmethoxycarbonyl (Fmoc) group, a benzyl group, an allyl group, and an allyloxycarbonyl (Alloc) group. The Cbz group can be deprotected by hydrofluoric acid, hydrogenation, or the like, the Boc group can be deprotected by trifluoroacetic acid (TFA), and the Fmoc group can be deprotected by a treatment with piperidine. The protection of an $\alpha$-carboxy group can be carried out using a methyl ester, an ethyl ester, a benzyl ester, a tert-butyl ester, a cyclohexyl ester, or the like. As for other functional groups of amino acids, a hydroxyl group of serine or threonine can be protected with a benzyl group or a tert-butyl group, and a hydroxyl group of tyrosine can be protected with a 2-bromobenzyloxycarbonyl group or a tert-butyl group. An amino group in a side chain of lysine and a carboxy group of glutamic acid or aspartic acid can be protected in the same manner as the $\alpha$-amino group and the $\alpha$-carboxy group. The activation of the carboxy group can be carried out using a condensing agent. Examples of the condensing agent include dicyclohexylcarbodiimide (DCC), diisopropylcarbodiimide (DIC), 1-ethyl-3-(3-dimethylaminopropyl) carbodiimide (EDC or WSC), (1H-benzotriazol-1-yloxy)tris(dimethylamino)phosphonium hexafluorophosphate (BOP), and 1-[bis(dimethylamino)methyl]-1H-benzotriazolium-3-oxide hexafluorophosphate (HBTU). Cleavage of a peptide chain from the resin can be carried out by a treatment with an acid such as TFA or hydrogen fluoride (HF).

Examples of the method for cyclization of the peptide include cyclization using an amide bond, a carbon-carbon bond, a thioether bond, a disulfide bond, an ester bond, a thioester bond, a lactam bond, a bond through a triazole structure, a bond through a fluorophore structure, and the like. The synthesis step and the cyclization reaction step of the peptide compound may be separate or may proceed consecutively. The cyclization can be carried out by methods known to those skilled in the art, for example, methods described in WO2013/100132, WO2008/117833, WO2012/074129, and the like. The cyclization portion is not limited, and may be any of a bond between an N-terminal and a C-terminal of a peptide, a bond between an N-terminal of a peptide and a side chain of another amino acid residue, a bond between a C-terminal of a peptide and a side chain of another amino acid residue, or a bond between side chains of amino acid residues, in which two or more of these bonds may be used in combination.

The method for thioether cyclization of a peptide is not particularly limited. For example, the peptide can be cyclized by including the following functional groups in a side chain or main chain of the peptide. The positions of functional groups 1 and 2 are not particularly limited, and either of functional groups 1 and 2 may be located at the N-terminal and C-terminal of the peptide, both of functional groups 1 and 2 may be located at the terminals, one of functional groups 1 and 2 may be terminal and the other of functional groups 1 and 2 may be non-terminal, or both of functional groups 1 and 2 may be non-terminal.

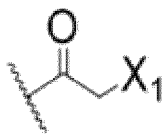 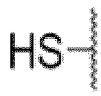

Functional group 1 Functional group 2

In the formula, $X_1$ represents chlorine, bromine, or iodine.

The synthesis step and the cyclization reaction step of the peptide compound may be separate or may proceed consecutively. The cyclization can be carried out by methods known to those skilled in the art, for example, methods described in WO2013/100132, WO2008/117833, WO2012/074129, and the like.

<Use applications of cyclic peptide>

The cyclic peptide can be used as a pharmaceutical product, a cosmetic product, a drug delivery system (DDS) material, and the like, without being limited thereto.

The present invention will be described with reference to the following examples, but the present invention is not limited thereto.

Examples

Structures of compound 1, compound 2, cyclosporin A, and isocyclosporin are shown below. The compound 1 and compound 2 are non-ionic in a physiological environment and contain a sulfur atom in the main chain structure of the cyclic peptide.

Compound 1

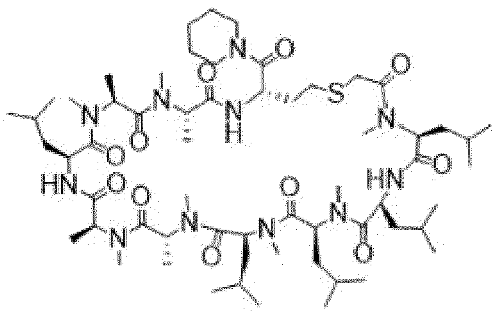

Compound 2

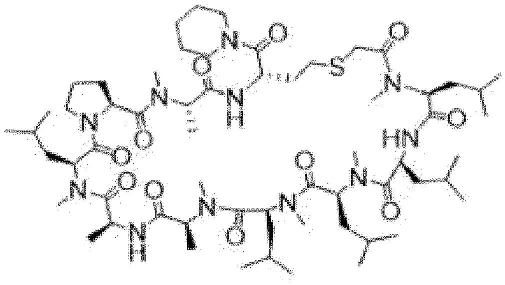

Cyclosporin A ( commercially available product, manufactur ed by FUJIFILM Wako Pure Chemical Corporation)

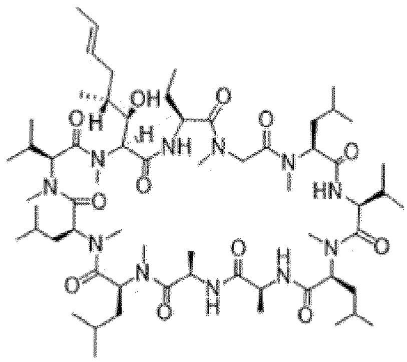

Isocyclosporin ( commercially available product, manufactured by FUJIFILM Wako Pure Chemical Corporation)

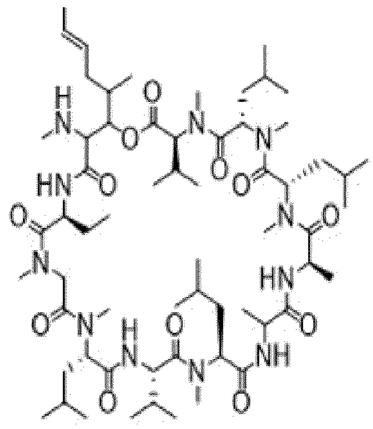

Example 1: Synthesis of Compound 1 and Compound 2

<Solid phase synthesis of peptide using automated peptide synthesizer>

The solid phase synthesis of a peptide was carried out using an automated peptide synthesizer (Syro I, manufactured by Biotage AB). The synthesis was carried out by setting a resin for solid phase synthesis, an N-methyl-2-pyrrolidone (NMP) solution of Fmoc amino acid (0.5 mol/L), an NMP solution of cyano-hydroxyimino-acetic acid ethyl ester (1 mol/L) and diisopropylethylamine (0.1 mol/L), an NMP solution of diisopropylcarbodiimide (1 mol/L), an NMP solution of piperidine (20% v/v), and an NMP solution of anhydrous acetic acid (20% v/v) in a peptide synthesizer. A cycle consisting of Fmoc deprotection (20 minutes), washing with NMP, condensation of Fmoc amino acids (1 hour), and washing with NMP as one cycle was repeated to elongate the peptide chain. After elongation of the peptide, the deprotection of the Fmoc group was carried out, and chloroacetic acid was condensed in the same manner as with amino acids.

<Cleavage from resin>

In order to cleave off a linear peptide from the resin, a solution of trifluoroacetic acid:triisopropylsilane:dichloromethane = 5:2.5:92.5 (mass ratio) corresponding to 5 times the amount of the resin was added to the resin, followed by shaking at room temperature for 2 hours. The reaction liquid was recovered by filtration. The reaction was further repeated once using the above solution of trifluoroacetic acid:triisopropylsilane:dichloromethane, and the reaction liquid was recovered by filtration. The recovered reaction liquids were all combined, the solvent was distilled off under reduced pressure, and the residue was thoroughly dried to obtain a crude purified product of a linear peptide.

<Cyclization reaction>

The crude purified product of the linear peptide was dissolved in acetonitrile (10 mL) and a solution (10 mL) of 0.1 mol/L TEAB (tetraethylammonium hydrogen carbonate) buffer:pure water = 1:9 (mass ratio), and the solution was adjusted to a pH of 8.5 $\pm$ 0.1. A solution (0.5 mol/L) of 1 molar equivalent of tris(2-carboxyethyl)phosphine (TCEP) was added thereto, followed by stirring at room temperature for 1 hour. After confirming the disappearance of the linear peptide as the raw material by LC/MS analysis (Acquity UPLC/SQD, manufactured by Waters Corporation), the solvent was distilled off under reduced pressure to obtain a crude purified product of a cyclic peptide.

<Purification of peptide>

The purification of the obtained crude purified product was carried out by liquid chromatography. Finally, a desired cyclic peptide was obtained as a freeze-dried powder.

Column: X Select CSH Prep C18 5 $\mu$m OBD (19 x 250 mm), manufactured by Waters Corporation
Column temperature: 40°C

Flow rate: 20 ml/min
Detection wavelength: 220 nm, 254 nm
Solvent: liquid A: 0.1% formic acid-water
liquid B: 0.1% formic acid-acetonitrile

Fmoc-amino acids were obtained from Watanabe Chemical Industries, Ltd.
N-methyl-2-pyrrolidone, diisopropylethylamine, diisopropylcarbodiimide, piperidine, and anhydrous acetic acid were obtained from FUJIFILM Wako Pure Chemical Corporation.
Ethyl cyanohydroxyiminoacetate was obtained from Tokyo Chemical Industry Co., Ltd.

<LC/MS analysis>

The mass spectrum (MS) was measured using an ACQUITY SQD LC/MS System (manufactured by Waters Corporation, ionization method: electrospray ionization (ESI) method).
Retention time (RT) was measured using an ACQUITY SQD LC/MS System (manufactured by Waters Corporation) and shown in minutes (min).

Column: BEH C18, 1.7 μm, 2.1 x 30 mm, manufactured by Waters Corporation
Solvent: liquid A: 0.1% formic acid-water
liquid B: 0.1% formic acid-acetonitrile
Gradient cycle: 0.00 min (liquid A/liquid B = 95/5), 2.00 min (liquid A/liquid B = 5/95), 3.00 min (liquid A/liquid B = 95/5)
Flow rate: 0.5 mL/min
Column temperature: room temperature
Detection wavelength: 254 nm

The measurement results of LC/MS of the compound 1 and the compound 2 are shown below.

[Table 1]

| | LC/MS analysis Observed MS (posi) | LC/MS analysis Retention Time (min) |
|---|---|---|
| Compound 1 | 1190.9 | 2.10 |
| Compound 2 | 1202.5 | 1.89 |

Example 2: Determination of r value from structure by MD calculation using restraint data of NMR measurement

The cyclic peptide was dissolved in DMSO-d6 to prepare a solution having a concentration of 5 mg/mL. A sample tube used was a SIGEMI tube (BMS-005B), and a sample volume was set to 400 μL. For 2D-NMR measurement (600 MHz Cryo system, manufactured by Bruker Corporation), the following three types of measurements were carried out for structure assignment: COSY (cosygpppgf, 128 integrations), TOCSY (melvphpp, 128 integrations, expansion time of 80 msec), and NOESY (noesygpphpp, 64 integrations, expansion time of 150 msec, 300 msec). The variable temperature $^1$H-NMR measurement (zg, a total of 64 times) was carried out at each of 25°C, 30°C, 35°C, 40°C, 45°C, and 50°C, and a ΔδNH/T (ppb/K) value was calculated from a change in chemical shift value depending on the temperature.
The chemical shift data of amide protons by variable temperature NMR and the distance data between amide groups (S: 1.8 to 2.7 Å, M: 1.8 to 3.5 Å, W: 1.8 to 5.0 Å) are shown in the tables below.

Compound 1:

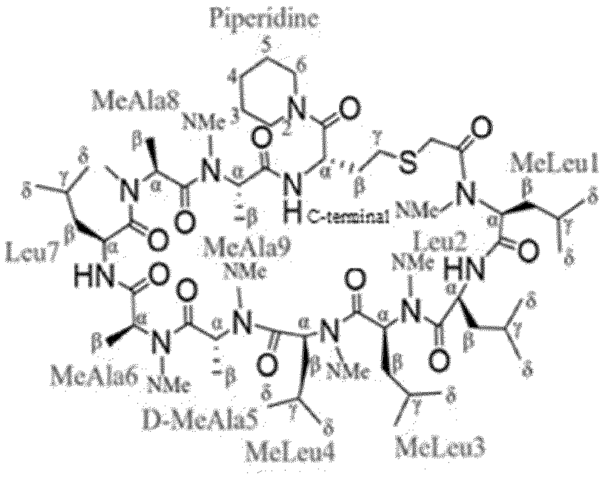

[Table 2]

| Compound 1: | | |
|---|---|---|
| Amide group A | Amide group B | S, M, W |
| Leu7 NH | MeAla6 NMe | M |
| C-terminal NH | MeAla9 NMe | W |
| Leu7 NH | Leu7 α | W |
| Leu7 NH | MeAla6 α | W |
| C-terminal NH | C-terminal γ | W |
| C-terminal NH | C-terminal α | W |
| C-terminal NH | MeAla9 α | M |
| Leu2 NH | MeLeul NMe | W |
| Leu2 NH | Leu2 α | W |
| Leu2 NH | MeLeul α | M |
| MeLeu3 α | MeLeu3 δ | M |
| MeLeu3 α | MeLeu4 NMe | S |
| MeLeu4 α | MeLeu4 δ | M |
| MeAla8 α | MeAla8 β | M |
| MeAla8 α | MeAla9 NMe | S |
| MeLeu4 α | MeAla5 NMe | S |
| MeAla6 α | MeAla6 β | M |
| MeAla6 α | MeAla6 NMe | W |
| MeAla9 α | MeAla9 β | M |
| MeLeu1 α | MeLeul δ | M |
| MeLeu1 α | MeLeu1 NMe | W |
| MeAla9 α | MeAla9 NMe | W |
| C-terminal α | C-terminal γ | W |
| C-terminal α | C-terminal β | W |

(continued)

| Compound 1: | | |
|---|---|---|
| Amide group A | Amide group B | S, M, W |
| C-terminal α | Piperidine 2,6 | S |
| Leu2 α | Leu2 δ | M |
| Leu2 α | MeLeu3 NMe | S |
| MeAla5 α | MeAla5 β | S |
| MeAla5 α | MeAla6 NMe | S |
| Leu7 α | Leu7 δ | M |
| Leu7 α | MeAla8 NMe | S |

[Table 3]

| | ΔδNH/T [ppb/K] |
|---|---|
| Leu2 NH | 1.3 |
| Ala7 NH | 3.8 |
| C-terminal NH | 2.7 |

Compound 2:

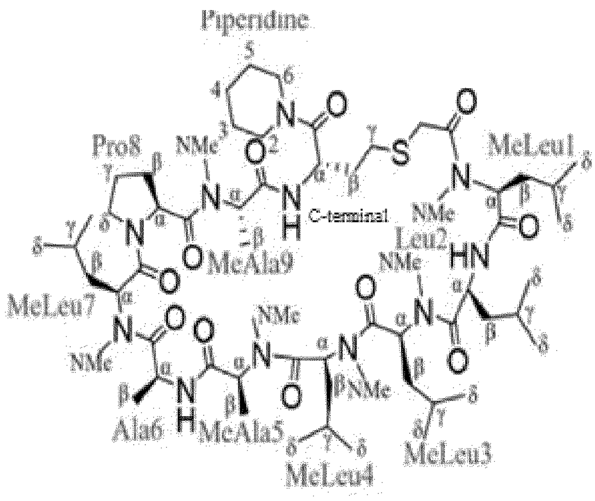

[Table 4]

| Compound 2: | | |
|---|---|---|
| Amide group A | Amide group B | S, M, W |
| Leu2 NH | MeLeul α | W |
| Leu2 NH | Leu2 α | W |
| Leu2 NH | Leu2 β | W |
| Leu2 NH | Leu2 γ | W |
| Ala6 NH | Ala6 α | W |
| Ala6 NH | Ala6 β | W |
| Ala6 NH | Piperidine 2, 6 | W |

(continued)

| Compound 2: | | |
|---|---|---|
| Amide group A | Amide group B | S, M, W |
| Ala6 NH | MeLeu5 NMe | W |
| Ala6 NH | MeLeu4 NMe | W |
| C-terminal NH | C-terminal α | W |
| C-terminal NH | C-terminal β | W |
| C-terminal NH | C-terminal γ | W |
| C-terminal NH | MeAla9 α | W |
| Leu2 α | MeLeu3 NMe | W |
| MeLeu3 α | MeLeu4 NMe | W |
| MeLeu4 α | MeLeu4 NMe | W |
| MeLeu4 α | MeLeu5 NMe | W |
| Ala6 α | MeLeu7 NMe | W |
| Ala6 α | Pro8 α | W |
| MeLeu7 α | Pro8 δ | W |

[Table 5]

| Variable temperature NMR | |
|---|---|
| | ΔδNH/T [ppb/K] |
| Leu2 NH | 1.2 |
| Ala6 NH | 0.3 |
| C-terminal NH | 1.8 |

Cyclosporin A

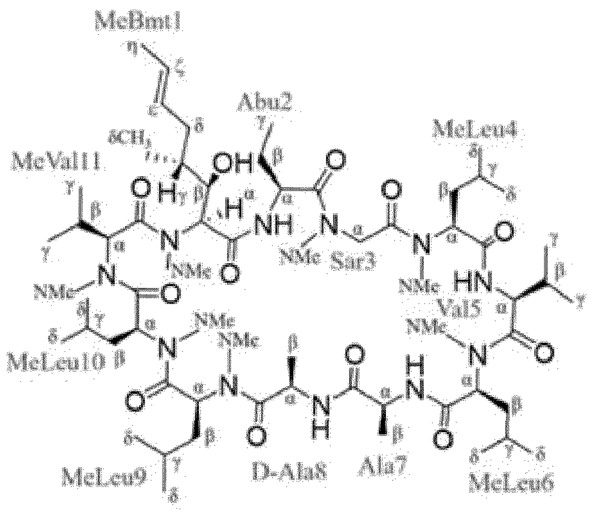

[Table 6]

| Cyclosporin A: | | |
|---|---|---|
| Amide group A | Amide group B | S, M, W |
| Abu2 NH | Abu2 γ | W |
| Abu2 NH | Abu2 β | W |
| MeBmtl β | Abu2 NH | W |
| Abu2 NH | Abu2 α | W |
| MeBmtl α | Abu2 NH | M |
| Ala7 NH | Ala7 β | M |
| Ala7 NH | MeVal11 NMe | W |
| Ala7 NH | Ala7 α | W |
| MeLeu6 α | Ala7 NH | S |
| MeBmtl α | Ala7 NH | W |
| Val5 NH | Val5 γ | W |
| Val5 NH | Val5 β | W |
| Val5 NH | MeLeu4 NMe | W |
| Val5 NH | Val5 α | S |
| MeLeu4 α | Val5 NH | W |
| D-Ala8 NH | D-Ala8 β | W |
| MeLeu6 g | D-Ala8 NH | W |
| D-Ala8 NH | MeVal11 NMe | W |
| Ala7 α | D-Ala8 NH | W |
| D-Ala8 NH | D-Ala8 α | W |
| MeLeu9 α | MeLeu9 δ | W |
| MeLeu9 α | MeLeu10 δ | W |
| MeLeu9 α | MeLeu9 β | W |
| MeLeu9 α | MeLeu 10 γ | W |
| MeBmt1α | MeBmtl δCH3 | W |
| MeBmtl α | MeBmtl δ | W |
| MeBmtl α | MeBmtl β | W |
| | | S, M, W |
| MeLeu4 α | MeLeu4 δ | M |
| MeLeu4 α | MeLeu4 γ | W |
| MeLeu4 α | MeLeu4 β | W |
| MeVal11 α | MeVal11 γ | W |
| MeVal11 α | MeVal11 β | W |
| MeVal11 α | MeBmt1 NMe | S |
| MeLeu10 α | MeLeu10 δ | M |
| MeLeu10 α | MeLeu10 β | W |
| MeLeu10 α | MeLeu10 γ | W |
| MeLeu10 α | MeLeu10 β | W |

(continued)

| Cyclosporin A: | | |
|---|---|---|
| Amide group A | Amide group B | S, M, W |
| MeLeu10 α | MeVal11 NCH3 | S |
| Abu2 α | Abu2 γ | W |
| Abu2 α | Abu2 β | M |
| Abu2 α | Sar3 NMe | S |
| MeLeu6 α | MeBmtl δ CH3 | W |
| MeLeu6 α | MeLeu6 δ | W |
| MeLeu6 α | MeLeu6 β | W |
| MeLeu6 α | MeLeu6 γ | W |
| D-Ala8 α | D-Ala8 β | W |
| D-Ala8 α | MeLeu9 NMe | S |
| Sar3 α | MeLeu4 NMe | S |
| Val5 α | Val5 γ | W |
| Val5 α | Val5 β | W |
| Val5 α | MeLeu6 NMe | S |
| Ala7 α | Ala7 β | M |

[Table 7]

| Variable temperature NMR | |
|---|---|
| | ΔδNH/T [ppb/K] |
| Abu2 NH | 3.5 |
| Val5 NH | 1.7 |
| Ala7 NH | 3.6 |
| D-Ala8 NH | 1.0 |

Next, the structure of the cyclic peptide was determined by restraining the structure generated by the molecular dynamics (MD) method using NMR data.

The calculation of the MD method was carried out using AmberTools 16. A GAFF force field was used for interactions, and RESP charges calculated by Gaussian 09 were used for charges. The NMR data (the HH distance) was used as the restraint condition using the NMR restraint option implemented in AmberTools 16. The procedure for calculating the structure of the cyclic peptide is as follows.

(1) 1,000 initial structures having different conformations are prepared for a linear peptide before cyclization of a target cyclic peptide.
Among the 1,000 initial structures, the initial structures of compound 1, compound 2, and cyclosporin A are shown in Fig. 1.
(2) Each linear initial structure is cyclized, and then the restraint based on the NMR data is applied at each step. The order is (i) cyclization/short-range HH distance, (ii) medium-range HH distance, and (iii) long-range HH distance, each of which is calculated over 0.2 ns.
(3) Among the 1,000 structures obtained, the structures are assigned priorities in order of satisfying the NMR data. The top 10 are drawn to determine the final structures.

Among the top 10 final structures, the structures of compound 1, compound 2, and cyclosporin A are shown in Fig. 2.

The final structures of compound 1, compound 2, and cyclosporin A are shown in Fig. 3.

In the structure having the highest priority, the three-dimensional coordinates of atoms belonging to the main chain of the cyclic peptide are represented by ($X_{a,1}$, $X_{a,2}$, $X_{a,3}$).

Here, a is a label that identifies the atoms belonging to the main chain, and takes an integer from 1 to N. N is the total number of atoms belonging to the main chain of the cyclic peptide.

The r value is calculated for the three-dimensional coordinates. The r value can be calculated according to the following procedure.

(1) Using three-dimensional coordinates as an input, the inertia tensor (a 3 × 3 matrix) is calculated according to the following expression.

$$\begin{pmatrix} I_{11} & I_{12} & I_{31} \\ I_{21} & I_{22} & I_{32} \\ I_{31} & I_{32} & I_{33} \end{pmatrix} = \begin{pmatrix} \sum_{a=1}^{N}\sum_{k=1}^{3}(X_{a,k})^2 - \sum_{a=1}^{N} X_{a,1}X_{a,1} & -\sum_{a=1}^{N} X_{a,2}X_{a,1} & -\sum_{a=1}^{N} X_{a,3}X_{a,1} \\ -\sum_{a=1}^{N} X_{a,2}X_{a,1} & \sum_{a=1}^{N}\sum_{k=1}^{3}(X_{a,k})^2 - \sum_{a=1}^{N} X_{a,2}X_{a,2} & -\sum_{a=1}^{N} X_{a,3}X_{a,2} \\ -\sum_{a=1}^{N} X_{a,3}X_{a,1} & -\sum_{a=1}^{N} X_{a,2}X_{a,3} & \sum_{a=1}^{N}\sum_{k=1}^{3}(X_{a,k})^2 - \sum_{a=1}^{N} X_{a,3}X_{a,3} \end{pmatrix}$$

(2) Eigenvalues of the inertia tensor are calculated. The obtained three eigenvalues are referred to as principal moments of inertia and are represented by ($I_1$, $I_2$, $I_3$).

(3) Using the principal moments of inertia as an input, each of axis lengths a, b, and c (a > b > c) of an ellipsoid with a uniform distribution is calculated according to the following expression.

$$a = \sqrt{\frac{5}{N}\left[\frac{\sum_{i=1}^{3} I_i}{2} - I_1\right]}$$

$$b = \sqrt{\frac{5}{N}\left[\frac{\sum_{i=1}^{3} I_i}{2} - I_2\right]}$$

$$c = \sqrt{\frac{5}{N}\left[\frac{\sum_{i=1}^{3} I_i}{2} - I_3\right]}$$

(4) Using each of axis lengths of the ellipsoid as an input, the molecular shape factor (r) is calculated according to the following expression.

$$r = \frac{2\sqrt{b^2 + c^2}}{\sqrt{c^2 + a^2} + \sqrt{a^2 + b^2}}$$

The coordinate data (X, Y, Z of each atom) for the final structure of the compound 1 are shown below.

[Table 8]

| a | atom_type | $X_{a,1}$ | $X_{a,2}$ | $X_{a,3}$ |
|---|---|---|---|---|
| 1 | H | -5.783 | 1.855 | 0.782 |
| 2 | H | -6.279 | 3.392 | 0.047 |
| 3 | H | -4.56 | 3.012 | 0.234 |
| 4 | H | -9.118 | -1.31 | -2.274 |
| 5 | H | -7.941 | -2.438 | -2.978 |
| 6 | H | -7.743 | -0.695 | -3.209 |
| 7 | H | -8.703 | -3.878 | -1.276 |
| 8 | H | -8.128 | -4.314 | 0.325 |
| 9 | H | -9.057 | -2.832 | 0.107 |
| 10 | H | -6.524 | -2.462 | 0.366 |
| 11 | H | -3.62 | 5.067 | -6.262 |
| 12 | H | -2 | 4.922 | -5.591 |
| 13 | H | -2.882 | 3.478 | -6.078 |
| 14 | H | -4.265 | 5.679 | -2.624 |
| 15 | H | -4.425 | 6.344 | -4.243 |
| 16 | H | -2.842 | 6.255 | -3.482 |
| 17 | H | -4.566 | 3.858 | -4.304 |
| 18 | H | -6.574 | 0.861 | -2.715 |
| 19 | H | -8.821 | 1.018 | -1.652 |
| 20 | H | -8.11 | 2.624 | -1.777 |
| 21 | H | -8.141 | 1.788 | -0.226 |
| 22 | H | 2.419 | -0.281 | 1.576 |
| 23 | H | 4.481 | -1.775 | 3.123 |
| 24 | H | 5.195 | -1.616 | 0.69 |
| 25 | H | 5.126 | 0.136 | 0.799 |
| 26 | H | 9.234 | 1.73 | 0.372 |
| 27 | H | 9.21 | 1.184 | 2.055 |
| 28 | H | 4.799 | -0.835 | 6.179 |
| 29 | H | 5.523 | -1.44 | 4.685 |
| 30 | H | 7.245 | -0.919 | 6.429 |
| 31 | H | 7.48 | 0.029 | 4.963 |
| 32 | H | 6.161 | 1.004 | 7.546 |
| 33 | H | 7.729 | 1.502 | 6.924 |

(continued)

| a | atom_type | $X_{a,1}$ | $X_{a,2}$ | $X_{a,3}$ |
|---|---|---|---|---|
| 34 | H | 5.929 | 3.183 | 6.391 |
| 35 | H | 6.669 | 2.52 | 4.932 |
| 36 | H | 3.985 | 1.68 | 6.16 |
| 37 | H | 4.229 | 2.537 | 4.619 |
| 38 | H | 7.031 | -1.731 | 2.311 |
| 39 | H | 6.87 | -0.013 | 2.702 |
| 40 | H | -3.714 | 3.948 | -1.471 |
| 41 | H | -1.746 | 3.955 | -3.117 |
| 42 | H | -2.45 | 2.534 | -3.883 |
| 43 | H | -0.775 | 1.389 | -2.058 |
| 44 | H | -2.383 | 0.652 | -2.156 |
| 45 | H | -1.46 | 0.566 | -0.646 |
| 46 | H | -0.851 | 1.57 | 1.069 |
| 47 | H | 0.275 | 3.14 | 2.716 |
| 48 | H | -0.666 | 4.473 | 2.055 |
| 49 | H | -2.785 | 3.266 | 2.68 |
| 50 | H | -0.897 | 1.364 | 4.167 |
| 51 | H | -1.957 | 0.929 | 2.831 |
| 52 | H | -2.643 | 1.427 | 4.37 |
| 53 | H | 0.034 | 4.863 | -0.09 |
| 54 | H | 0.672 | 4.133 | -1.569 |
| 55 | H | 1.763 | 4.495 | -0.219 |
| 56 | H | 2.565 | 2.729 | -1.578 |
| 57 | H | 3.748 | 1.964 | 1.063 |
| 58 | H | 4.127 | 0.671 | -1.775 |
| 59 | H | 2.998 | -0.188 | -0.734 |
| 60 | H | 1.152 | 0.471 | -2.441 |
| 61 | H | 2.489 | 2.178 | -3.7 |
| 62 | H | 1.909 | 0.896 | -4.758 |
| 63 | H | 3.59 | 0.918 | -4.241 |
| 64 | H | 1.732 | -1.467 | -3.93 |
| 65 | H | 1.909 | -1.844 | -2.22 |
| 66 | H | 3.336 | -1.528 | -3.214 |
| 67 | H | 5.343 | 3.504 | 1.857 |
| 68 | H | 7.087 | 5.392 | 1.089 |
| 69 | H | 5.94 | 5.667 | -0.22 |
| 70 | H | 6.591 | 3.484 | -1.511 |
| 71 | H | 7.349 | 1.898 | -1.273 |
| 72 | H | 8.277 | 3.378 | -0.97 |

(continued)

| a | atom_type | $X_{a,1}$ | $X_{a,2}$ | $X_{a,3}$ |
|---|---|---|---|---|
| 73 | H | -4.387 | -2.698 | -0.471 |
| 74 | H | -4.081 | -4.739 | -2.597 |
| 75 | H | -2.712 | -1.983 | -2.502 |
| 76 | H | -2.136 | -3.277 | -3.53 |
| 77 | H | -4.958 | -2.094 | -3.656 |
| 78 | H | -4.157 | -0.962 | -5.748 |
| 79 | H | -2.517 | -1.496 | -5.404 |
| 80 | H | -3.338 | -0.381 | -4.306 |
| 81 | H | -5.063 | -3.244 | -5.886 |
| 82 | H | -4.944 | -4.372 | -4.544 |
| 83 | H | -3.529 | -4.034 | -5.546 |
| 84 | H | -0.989 | -5.873 | 0.441 |
| 85 | H | -2.32 | -5.611 | -3.408 |
| 86 | H | -1.117 | -6.787 | -2.885 |
| 87 | H | -0.603 | -5.164 | -3.378 |
| 88 | H | -2.618 | 3.827 | 5.097 |
| 89 | H | -1.838 | 5.057 | 4.107 |
| 90 | H | -0.863 | 3.882 | 4.983 |
| 91 | H | 1.277 | -6.691 | -0.11 |
| 92 | H | 1.09 | -6.223 | -1.794 |
| 93 | H | 0.092 | -7.515 | -1.115 |
| 94 | H | 0.917 | -1.893 | 0.386 |
| 95 | H | 0.601 | -5.677 | 2.039 |
| 96 | H | -0.829 | -4.722 | 2.487 |
| 97 | H | 0.78 | -4.285 | 3.104 |
| 98 | H | 0.123 | -0.617 | 2.335 |
| 99 | H | -0.109 | -2.089 | 3.282 |
| 100 | H | -1.113 | -1.785 | 1.865 |
| 101 | H | 5.296 | 5.772 | 2.775 |
| 102 | H | 6.78 | 7.634 | 2.059 |
| 103 | H | 5.175 | 8.234 | 2.465 |
| 104 | H | 5.652 | 8.069 | 0.78 |
| 105 | H | 3.55 | 6.479 | 0.362 |
| 106 | H | 3.142 | 6.836 | 2.039 |
| 107 | H | 3.281 | 5.166 | 1.505 |
| 108 | C | -5.534 | 2.583 | 0.005 |
| 109 | C | -8.05 | -1.463 | -2.497 |
| 110 | C | -8.308 | -3.478 | -0.346 |
| 111 | O | -5.646 | -0.465 | 0.03 |

(continued)

| a | atom_type | $X_{a,1}$ | $X_{a,2}$ | $X_{a,3}$ |
|---|---|---|---|---|
| 112 | O | -6.406 | -4.306 | -2.364 |
| 113 | C | -7.001 | -2.722 | -0.592 |
| 114 | N | -7.23 | -1.422 | -1.293 |
| 115 | C | -6.464 | -0.347 | -0.904 |
| 116 | C | -2.985 | 4.459 | -5.621 |
| 117 | C | -3.791 | 5.736 | -3.601 |
| 118 | C | -3.584 | 4.344 | -4.213 |
| 119 | C | -6.649 | 1.019 | -1.625 |
| 120 | C | -8.012 | 1.652 | -1.297 |
| 121 | N | 2.927 | -1.095 | 1.947 |
| 122 | C | 4.265 | -0.883 | 2.513 |
| 123 | C | 4.223 | 0.427 | 3.335 |
| 124 | C | 5.328 | -0.773 | 1.374 |
| 125 | S | 7.983 | -0.319 | 0.575 |
| 126 | C | 8.596 | 1.328 | 1.162 |
| 127 | N | 4.775 | 0.53 | 4.575 |
| 128 | O | 3.646 | 1.42 | 2.813 |
| 129 | C | 5.43 | -0.551 | 5.317 |
| 130 | C | 6.815 | -0.117 | 5.821 |
| 131 | C | 6.727 | 1.181 | 6.624 |
| 132 | C | 6.046 | 2.272 | 5.795 |
| 133 | C | 4.672 | 1.807 | 5.302 |
| 134 | C | -4.534 | 2.061 | -2.235 |
| 135 | N | -5.527 | 1.93 | -1.298 |
| 136 | O | -4.586 | 1.407 | -3.299 |
| 137 | C | 6.761 | -0.751 | 1.902 |
| 138 | C | -3.355 | 3.044 | -1.988 |
| 139 | N | -2.35 | 2.437 | -1.07 |
| 140 | C | -2.691 | 3.444 | -3.328 |
| 141 | C | -1.706 | 1.201 | -1.5 |
| 142 | C | -1.971 | 3.154 | 0.041 |
| 143 | C | -0.772 | 2.654 | 0.889 |
| 144 | O | -2.578 | 4.199 | 0.351 |
| 145 | N | 0.484 | 2.832 | 0.106 |
| 146 | C | -0.678 | 3.395 | 2.244 |
| 147 | C | -1.841 | 3.076 | 3.211 |
| 148 | C | -1.832 | 1.612 | 3.669 |
| 149 | C | 1.335 | 1.769 | -0.026 |
| 150 | C | 0.757 | 4.145 | -0.473 |

(continued)

| a | atom_type | $X_{a,1}$ | $X_{a,2}$ | $X_{a,3}$ |
|---|---|---|---|---|
| 151 | C | 2.659 | 1.946 | -0.808 |
| 152 | O | 1.058 | 0.665 | 0.501 |
| 153 | N | 3.691 | 2.419 | 0.136 |
| 154 | C | 3.076 | 0.611 | -1.477 |
| 155 | C | 2.201 | 0.257 | -2.703 |
| 156 | C | 2.569 | 1.115 | -3.921 |
| 157 | C | 2.302 | -1.238 | -3.033 |
| 158 | C | 4.491 | 3.488 | -0.168 |
| 159 | C | 5.635 | 3.802 | 0.837 |
| 160 | N | 6.797 | 2.932 | 0.493 |
| 161 | O | 4.356 | 4.133 | -1.223 |
| 162 | C | 6.033 | 5.3 | 0.807 |
| 163 | C | 7.28 | 2.921 | -0.883 |
| 164 | C | 7.428 | 2.246 | 1.502 |
| 165 | O | 7.031 | 2.33 | 2.684 |
| 166 | C | -6.007 | -3.54 | -1.47 |
| 167 | N | -4.685 | -3.282 | -1.252 |
| 168 | C | -3.621 | -3.897 | -2.052 |
| 169 | C | -2.535 | -4.4 | -1.075 |
| 170 | O | -2.554 | -4.013 | 0.113 |
| 171 | C | -3.035 | -2.865 | -3.063 |
| 172 | C | -4.038 | -2.432 | -4.158 |
| 173 | C | -3.475 | -1.246 | -4.951 |
| 174 | C | -4.413 | -3.594 | -5.087 |
| 175 | N | -1.559 | -5.277 | -1.484 |
| 176 | C | -0.485 | -5.513 | -0.474 |
| 177 | C | -1.388 | -5.726 | -2.854 |
| 178 | C | -1.786 | 4.018 | 4.422 |
| 179 | C | 0.551 | -6.549 | -0.908 |
| 180 | C | 0.189 | -4.133 | -0.189 |
| 181 | O | 0.441 | -3.371 | -1.145 |
| 182 | N | 0.494 | -3.788 | 1.1 |
| 183 | C | 0.909 | -2.394 | 1.368 |
| 184 | C | 0.241 | -4.662 | 2.237 |
| 185 | C | 2.333 | -2.332 | 1.969 |
| 186 | C | -0.112 | -1.677 | 2.274 |
| 187 | O | 2.91 | -3.322 | 2.451 |
| 188 | C | 5.191 | 6.18 | 1.756 |
| 189 | C | 5.733 | 7.615 | 1.765 |

(continued)

| a | atom_type | $X_{a,1}$ | $X_{a,2}$ | $X_{a,3}$ |
|---|---|---|---|---|
| 190 | C | 3.702 | 6.164 | 1.392 |

The coordinate data of atoms (X, Y, Z of each atom) of the main chain of the compound 1 are shown below.

[Table 9]

| a | atom_type | $X_{a,1}$ | $X_{a,2}$ | $X_{a,3}$ |
|---|---|---|---|---|
| 135 | N | -5.527 | 1.93 | -1.298 |
| 119 | C | -6.649 | 1.019 | -1.625 |
| 115 | C | -6.464 | -0.347 | -0.904 |
| 114 | N | -7.23 | -1.422 | -1.293 |
| 113 | C | -7.001 | -2.722 | -0.592 |
| 166 | C | -6.007 | -3.54 | -1.47 |
| 167 | N | -4.685 | -3.282 | -1.252 |
| 168 | C | -3.621 | -3.897 | -2.052 |
| 169 | C | -2.535 | -4.4 | -1.075 |
| 175 | N | -1.559 | -5.277 | -1.484 |
| 176 | C | -0.485 | -5.513 | -0.474 |
| 180 | C | 0.189 | -4.133 | -0.189 |
| 182 | N | 0.494 | -3.788 | 1.1 |
| 183 | C | 0.909 | -2.394 | 1.368 |
| 185 | C | 2.333 | -2.332 | 1.969 |
| 121 | N | 2.927 | -1.095 | 1.947 |
| 122 | C | 4.265 | -0.883 | 2.513 |
| 124 | C | 5.328 | -0.773 | 1.374 |
| 137 | C | 6.761 | -0.751 | 1.902 |
| 125 | S | 7.983 | -0.319 | 0.575 |
| 126 | C | 8.596 | 1.328 | 1.162 |
| 164 | C | 7.428 | 2.246 | 1.502 |
| 160 | N | 6.797 | 2.932 | 0.493 |
| 159 | C | 5.635 | 3.802 | 0.837 |
| 158 | C | 4.491 | 3.488 | -0.168 |
| 153 | N | 3.691 | 2.419 | 0.136 |
| 151 | C | 2.659 | 1.946 | -0.808 |
| 149 | C | 1.335 | 1.769 | -0.026 |
| 145 | N | 0.484 | 2.832 | 0.106 |
| 143 | C | -0.772 | 2.654 | 0.889 |
| 142 | C | -1.971 | 3.154 | 0.041 |
| 139 | N | -2.35 | 2.437 | -1.07 |
| 138 | C | -3.355 | 3.044 | -1.988 |
| 134 | C | -4.534 | 2.061 | -2.235 |

The values of all the components (3 x 3) of the inertia tensor for the compound 1 are shown below.

[Table 10]

| | | | | | | |
|---|---|---|---|---|---|---|
| $I_{11}$ | $I_{21}$ | $I_{31}$ | | 331.3524 | -129.1 | -158.085 |
| $I_{12}$ | $I_{22}$ | $I_{32}$ | | -129.1 | 816.8712 | -10.5221 |
| $I_{13}$ | $I_{23}$ | $I_{33}$ | | -158.085 | -10.5221 | 1032.943 |

The values of all the components (3) of the principal moments of inertia for the compound 1 are shown below.
$I_1$ = 267.343, $I_2$ = 845.672, $I_3$ = 1068.152
The values of a, b, and c for the compound 1 are shown below.
a = 11.00294, b = 6.001367, c = 1.816242
The r value for the compound 1 is shown below.
r value: 0.529463
An ellipsoid diagram for the compound 1 is shown in Fig. 4.
The coordinate data (X, Y, Z of each atom) for the final structure of the compound 2 are shown below.

[Table 11]

| a | atom_type | $X_{a,1}$ | $X_{a,2}$ | $X_{a,3}$ |
|---|---|---|---|---|
| 1 | N | 1.698 | 5.023 | -4.054 |
| 2 | C | 0.698 | 5.763 | -4.64 |
| 3 | C | 2.308 | 3.826 | -4.687 |
| 4 | C | 0.254 | 7.032 | -3.893 |
| 5 | O | 0.174 | 5.419 | -5.681 |
| 6 | C | 3.815 | 4.079 | -4.97 |
| 7 | C | 2.077 | 2.562 | -3.811 |
| 8 | N | -0.146 | 6.738 | -2.474 |
| 9 | C | 1.301 | 8.19 | -4.015 |
| 10 | C | 4.1 | 5.2 | -6 |
| 11 | O | 2.116 | 2.611 | -2.593 |
| 12 | N | 1.679 | 1.4 | -4.478 |
| 13 | C | -1.62 | 6.637 | -2.293 |
| 14 | C | 0.768 | 6.551 | -1.412 |
| 15 | C | 1.572 | 8.654 | -5.475 |
| 16 | C | 5.615 | 5.418 | -6.133 |
| 17 | C | 3.507 | 4.882 | -7.384 |
| 18 | C | 0.662 | 0.512 | -3.844 |
| 19 | C | 1.971 | 1.14 | -5.914 |
| 20 | O | 1.973 | 6.49 | -1.588 |
| 21 | C | 0.185 | 6.452 | 0.011 |
| 22 | C | 0.319 | 9.273 | -6.13 |
| 23 | C | 2.724 | 9.676 | -5.5 |
| 24 | C | -0.778 | 0.801 | -4.386 |
| 25 | C | 1.064 | -0.978 | -3.993 |
| 26 | S | -0.252 | 4.705 | 0.462 |
| 27 | C | -1.274 | 2.261 | -4.218 |

(continued)

| a | atom_type | $X_{a,1}$ | $X_{a,2}$ | $X_{a,3}$ |
|---|---|---|---|---|
| 28 | O | 0.432 | -1.739 | -4.707 |
| 29 | C | 1.429 | 3.956 | 0.436 |
| 30 | C | -2.632 | 2.44 | -4.913 |
| 31 | C | -1.423 | 2.662 | -2.74 |
| 32 | C | 1.335 | 2.459 | 0.763 |
| 33 | C | 0.851 | 2.168 | 2.217 |
| 34 | C | 0.769 | 0.643 | 2.539 |
| 35 | N | 1.757 | 2.816 | 3.182 |
| 36 | O | 1.485 | 0.147 | 3.397 |
| 37 | N | -0.086 | -0.15 | 1.762 |
| 38 | C | 1.378 | 3.051 | 4.494 |
| 39 | C | -1.305 | 0.351 | 1.075 |
| 40 | C | -0.013 | -1.633 | 1.827 |
| 41 | O | 0.203 | 3.05 | 4.818 |
| 42 | C | 2.526 | 3.25 | 5.528 |
| 43 | C | -1.459 | -0.251 | -0.342 |
| 44 | C | -0.146 | -2.293 | 0.435 |
| 45 | N | 3.793 | 3.682 | 4.876 |
| 46 | C | 2.15 | 4.16 | 6.736 |
| 47 | C | -1.403 | -1.794 | -0.314 |
| 48 | C | 3.865 | 5.109 | 4.451 |
| 49 | C | 4.935 | 2.869 | 4.826 |
| 50 | C | 4.711 | 1.351 | 4.597 |
| 51 | O | 6.058 | 3.347 | 4.823 |
| 52 | N | 5.468 | 0.924 | 3.416 |
| 53 | C | 5.227 | 0.494 | 5.774 |
| 54 | C | 6.554 | 0.001 | 3.788 |
| 55 | C | 5.181 | 1.413 | 2.145 |
| 56 | C | 6.611 | -0.007 | 5.325 |
| 57 | C | 5.681 | 0.563 | 0.951 |
| 58 | O | 4.432 | 2.365 | 1.988 |
| 59 | N | 5.111 | -0.816 | 1.023 |
| 60 | C | 5.405 | 1.205 | -0.442 |
| 61 | C | 5.932 | -1.947 | 0.899 |
| 62 | C | 3.649 | -0.905 | 1.267 |
| 63 | C | 6.215 | 2.504 | -0.698 |
| 64 | C | 5.268 | -3.345 | 1.2 |
| 65 | O | 7.12 | -1.833 | 0.635 |
| 66 | C | 7.692 | 2.196 | -1.016 |

(continued)

| a | atom_type | $X_{a,1}$ | $X_{a,2}$ | $X_{a,3}$ |
|---|---|---|---|---|
| 67 | C | 5.599 | 3.301 | -1.858 |
| 68 | N | 4.411 | -3.775 | 0.098 |
| 69 | C | 6.263 | -4.496 | 1.452 |
| 70 | C | 3.133 | -4.242 | 0.267 |
| 71 | C | 2.526 | -5.03 | -0.908 |
| 72 | O | 2.512 | -4.035 | 1.29 |
| 73 | N | 2.325 | -4.158 | -2.107 |
| 74 | C | 3.311 | -6.334 | -1.225 |
| 75 | C | 3.391 | -3.608 | -2.852 |
| 76 | C | 0.914 | -4.094 | -2.572 |
| 77 | C | 3.038 | -2.529 | -3.923 |
| 78 | O | 4.555 | -3.915 | -2.648 |
| 79 | N | 2.252 | -1.382 | -3.369 |
| 80 | C | 4.236 | -2.056 | -4.821 |
| 81 | C | 2.76 | -0.737 | -2.131 |
| 82 | C | 5.394 | -1.305 | -4.098 |
| 83 | C | 6.759 | -1.889 | -4.499 |
| 84 | C | 5.375 | 0.197 | -4.443 |
| 85 | H | 2.039 | 5.346 | -3.153 |
| 86 | H | 1.792 | 3.639 | -5.636 |
| 87 | H | -0.641 | 7.35 | -4.439 |
| 88 | H | 4.299 | 4.354 | -4.027 |
| 89 | H | 4.297 | 3.155 | -5.309 |
| 90 | H | 0.962 | 9.05 | -3.424 |
| 91 | H | 2.26 | 7.877 | -3.586 |
| 92 | H | 3.66 | 6.138 | -5.641 |
| 93 | H | -2.022 | 7.602 | -1.959 |
| 94 | H | -2.108 | 6.352 | -3.234 |
| 95 | H | -1.867 | 5.867 | -1.549 |
| 96 | H | 1.882 | 7.79 | -6.076 |
| 97 | H | 6.112 | 4.517 | -6.515 |
| 98 | H | 5.826 | 6.244 | -6.822 |
| 99 | H | 6.058 | 5.671 | -5.162 |
| 100 | H | 3.83 | 5.629 | -8.12 |
| 101 | H | 3.828 | 3.895 | -7.737 |
| 102 | H | 2.411 | 4.91 | -7.36 |
| 103 | H | 0.651 | 0.702 | -2.766 |
| 104 | H | 1.902 | 0.069 | -6.139 |
| 105 | H | 1.248 | 1.673 | -6.544 |

(continued)

| a | atom_type | $X_{a,1}$ | $X_{a,2}$ | $X_{a,3}$ |
|---|---|---|---|---|
| 106 | H | 2.986 | 1.474 | -6.161 |
| 107 | H | 0.942 | 6.822 | 0.713 |
| 108 | H | -0.699 | 7.094 | 0.097 |
| 109 | H | 0.571 | 9.711 | -7.104 |
| 110 | H | -0.451 | 8.512 | -6.308 |
| 111 | H | -0.107 | 10.064 | -5.501 |
| 112 | H | 2.467 | 10.573 | -4.924 |
| 113 | H | 3.638 | 9.243 | -5.075 |
| 114 | H | 2.947 | 9.983 | -6.53 |
| 115 | H | -0.812 | 0.537 | -5.451 |
| 116 | H | -1.485 | 0.126 | -3.888 |
| 117 | H | -0.563 | 2.943 | -4.701 |
| 118 | H | 2.068 | 4.473 | 1.161 |
| 119 | H | 1.858 | 4.087 | -0.561 |
| 120 | H | -2.971 | 3.481 | -4.834 |
| 121 | H | -2.556 | 2.196 | -5.98 |
| 122 | H | -3.395 | 1.792 | -4.465 |
| 123 | H | -1.789 | 3.692 | -2.662 |
| 124 | H | -2.139 | 2.012 | -2.226 |
| 125 | H | -0.466 | 2.621 | -2.208 |
| 126 | H | 2.327 | 2.029 | 0.607 |
| 127 | H | 0.683 | 1.991 | 0.02 |
| 128 | H | -0.159 | 2.578 | 2.37 |
| 129 | H | 2.742 | 2.883 | 2.939 |
| 130 | H | -1.298 | 1.443 | 0.997 |
| 131 | H | -2.178 | 0.072 | 1.678 |
| 132 | H | 0.941 | -1.947 | 2.269 |
| 133 | H | -0.819 | -1.994 | 2.478 |
| 134 | H | 2.682 | 2.251 | 5.951 |
| 135 | H | -0.656 | 0.128 | -0.985 |
| 136 | H | -2.409 | 0.077 | -0.782 |
| 137 | H | 0.747 | -2.064 | -0.158 |
| 138 | H | -0.187 | -3.384 | 0.542 |
| 139 | H | 1.868 | 5.164 | 6.397 |
| 140 | H | 1.291 | 3.742 | 7.276 |
| 141 | H | 2.99 | 4.252 | 7.435 |
| 142 | H | -2.301 | -2.181 | 0.186 |
| 143 | H | -1.413 | -2.185 | -1.338 |
| 144 | H | 2.879 | 5.482 | 4.149 |

(continued)

| a | atom_type | $X_{a,1}$ | $X_{a,2}$ | $X_{a,3}$ |
|---|---|---|---|---|
| 145 | H | 4.258 | 5.718 | 5.276 |
| 146 | H | 4.557 | 5.198 | 3.601 |
| 147 | H | 3.655 | 1.134 | 4.411 |
| 148 | H | 5.279 | 1.062 | 6.713 |
| 149 | H | 4.558 | -0.363 | 5.926 |
| 150 | H | 7.51 | 0.335 | 3.362 |
| 151 | H | 6.321 | -1.006 | 3.422 |
| 152 | H | 7.384 | 0.693 | 5.669 |
| 153 | H | 6.843 | -1.003 | 5.724 |
| 154 | H | 6.768 | 0.496 | 1.079 |
| 155 | H | 5.641 | 0.478 | -1.229 |
| 156 | H | 4.334 | 1.42 | -0.534 |
| 157 | H | 3.461 | -1.301 | 2.273 |
| 158 | H | 3.18 | 0.079 | 1.181 |
| 159 | H | 3.178 | -1.562 | 0.53 |
| 160 | H | 6.182 | 3.142 | 0.195 |
| 161 | H | 4.666 | -3.205 | 2.11 |
| 162 | H | 7.778 | 1.544 | -1.895 |
| 163 | H | 8.243 | 3.121 | -1.222 |
| 164 | H | 8.186 | 1.698 | -0.173 |
| 165 | H | 6.162 | 4.226 | -2.034 |
| 166 | H | 5.601 | 2.712 | -2.781 |
| 167 | H | 4.563 | 3.579 | -1.624 |
| 168 | H | 4.851 | -3.945 | -0.804 |
| 169 | H | 5.724 | -5.424 | 1.679 |
| 170 | H | 6.891 | -4.656 | 0.568 |
| 171 | H | 6.921 | -4.254 | 2.294 |
| 172 | H | 1.544 | -5.347 | -0.538 |
| 173 | H | 2.846 | -6.874 | -2.059 |
| 174 | H | 4.358 | -6.137 | -1.485 |
| 175 | H | 3.31 | -6.996 | -0.35 |
| 176 | H | 0.3 | -4.864 | -2.089 |
| 177 | H | 0.491 | -3.111 | -2.336 |
| 178 | H | 0.861 | -4.256 | -3.655 |
| 179 | H | 2.379 | -3.073 | -4.611 |
| 180 | H | 4.647 | -2.948 | -5.312 |
| 181 | H | 3.846 | -1.435 | -5.636 |
| 182 | H | 1.942 | -0.608 | -1.412 |
| 183 | H | 3.542 | -1.344 | -1.66 |

(continued)

| a | atom_type | $X_{a,1}$ | $X_{a,2}$ | $X_{a,3}$ |
|---|---|---|---|---|
| 184 | H | 3.179 | 0.248 | -2.363 |
| 185 | H | 5.299 | -1.409 | -3.013 |
| 186 | H | 7.572 | -1.361 | -3.985 |
| 187 | H | 6.819 | -2.947 | -4.213 |
| 188 | H | 6.922 | -1.811 | -5.58 |
| 189 | H | 6.187 | 0.716 | -3.919 |
| 190 | H | 5.511 | 0.353 | -5.52 |
| 191 | H | 4.428 | 0.655 | -4.144 |

The coordinate data of atoms (X, Y, Z of each atom) of the main chain of the compound 2 are shown below.

[Table 12]

| a | atom_type | $X_{a,1}$ | $X_{a,2}$ | $X_{a,3}$ |
|---|---|---|---|---|
| 1 | N | 1.698 | 5.023 | -4.054 |
| 3 | C | 2.308 | 3.826 | -4.687 |
| 7 | C | 2.077 | 2.562 | -3.811 |
| 12 | N | 1.679 | 1.4 | -4.478 |
| 18 | C | 0.662 | 0.512 | -3.844 |
| 25 | C | 1.064 | -0.978 | -3.993 |
| 79 | N | 2.252 | -1.382 | -3.369 |
| 77 | C | 3.038 | -2.529 | -3.923 |
| 75 | C | 3.391 | -3.608 | -2.852 |
| 73 | N | 2.325 | -4.158 | -2.107 |
| 71 | C | 2.526 | -5.03 | -0.908 |
| 70 | C | 3.133 | -4.242 | 0.267 |
| 68 | N | 4.411 | -3.775 | 0.098 |
| 64 | C | 5.268 | -3.345 | 1.2 |
| 61 | C | 5.932 | -1.947 | 0.899 |
| 59 | N | 5.111 | -0.816 | 1.023 |
| 57 | C | 5.681 | 0.563 | 0.951 |
| 55 | C | 5.181 | 1.413 | 2.145 |
| 52 | N | 5.468 | 0.924 | 3.416 |
| 50 | C | 4.711 | 1.351 | 4.597 |
| 49 | C | 4.935 | 2.869 | 4.826 |
| 45 | N | 3.793 | 3.682 | 4.876 |
| 42 | C | 2.526 | 3.25 | 5.528 |
| 38 | C | 1.378 | 3.051 | 4.494 |
| 35 | N | 1.757 | 2.816 | 3.182 |
| 33 | C | 0.851 | 2.168 | 2.217 |
| 32 | C | 1.335 | 2.459 | 0.763 |

(continued)

| a | atom_type | $X_{a,1}$ | $X_{a,2}$ | $X_{a,3}$ |
|---|---|---|---|---|
| 29 | C | 1.429 | 3.956 | 0.436 |
| 26 | S | -0.252 | 4.705 | 0.462 |
| 21 | C | 0.185 | 6.452 | 0.011 |
| 14 | C | 0.768 | 6.551 | -1.412 |
| 8 | N | -0.146 | 6.738 | -2.474 |
| 4 | C | 0.254 | 7.032 | -3.893 |
| 2 | C | 0.698 | 5.763 | -4.64 |

The values of all the components (3 x 3) of the inertia tensor for the compound 2 are shown below.

[Table 13]

| | | | | | | |
|---|---|---|---|---|---|---|
| $I_{11}$ | $I_{21}$ | $I_{31}$ | | 753.2114 | 117.8032 | -88.5084 |
| $I_{12}$ | $I_{22}$ | $I_{32}$ | | 117.8032 | 456.9073 | -6.27265 |
| $I_{13}$ | $I_{23}$ | $I_{33}$ | | -88.5084 | -6.27265 | 526.609 |

The values of all the components (3) of the principal moments of inertia for the compound 2 are shown below.
$I_1$ = 410.2238, $I_2$ = 507.0146, $I_3$ = 819.4893

The values of a, b, and c for the compound 2 are shown below.
a = 8.208138, b = 7.289691, c = 2.680939

The r value for the compound 2 is shown below.
r value: 0.792042

An ellipsoid diagram for the compound 2 is shown in Fig. 5.

The coordinate data (X, Y, Z of each atom) for the final structure of cyclosporin A are shown below.

[Table 14]

| a | atom_type | $X_{a,1}$ | $X_{a,2}$ | $X_{a,3}$ |
|---|---|---|---|---|
| 1 | N | 1.43 | -0.813 | -2.378 |
| 2 | C | 1.642 | -2.092 | -3.076 |
| 3 | C | 1.486 | -1.878 | -4.585 |
| 4 | C | 0.611 | -3.168 | -2.61 |
| 5 | O | -0.615 | -2.96 | -2.771 |
| 6 | N | 1.053 | -4.355 | -2.093 |
| 7 | C | 2.432 | -4.586 | -1.666 |
| 8 | C | 0.084 | -5.48 | -1.944 |
| 9 | C | 0.585 | -6.717 | -2.732 |
| 10 | C | -0.469 | -7.843 | -2.845 |
| 11 | C | -1.603 | -7.474 | -3.81 |
| 12 | C | 0.2 | -9.154 | -3.278 |
| 13 | C | -0.076 | -5.848 | -0.44 |
| 14 | O | 0.779 | -6.579 | 0.095 |
| 15 | N | -1.132 | -5.359 | 0.299 |
| 16 | C | -1.304 | -5.859 | 1.663 |
| 17 | C | -2.213 | -4.49 | -0.237 |

(continued)

| a | atom_type | $X_{a,1}$ | $X_{a,2}$ | $X_{a,3}$ |
|---|---|---|---|---|
| 18 | C | -3.409 | -5.339 | -0.745 |
| 19 | C | -4.416 | -4.527 | -1.592 |
| 20 | C | -3.861 | -4.195 | -2.985 |
| 21 | C | -5.747 | -5.283 | -1.708 |
| 22 | C | -2.674 | -3.519 | 0.893 |
| 23 | O | -3.758 | -3.687 | 1.485 |
| 24 | N | -1.838 | -2.487 | 1.246 |
| 25 | C | -0.624 | -2.172 | 0.504 |
| 26 | C | -2.251 | -1.613 | 2.374 |
| 27 | C | -1.094 | -1.335 | 3.39 |
| 28 | C | -1.627 | -0.51 | 4.569 |
| 29 | C | -0.452 | -2.636 | 3.883 |
| 30 | C | -2.805 | -0.261 | 1.849 |
| 31 | O | -2.015 | 0.566 | 1.309 |
| 32 | N | -4.128 | 0.043 | 1.993 |
| 33 | C | -5.065 | -0.883 | 2.636 |
| 34 | C | -4.513 | 1.473 | 1.754 |
| 35 | C | -4.16 | 2.294 | 3.017 |
| 36 | O | -4.771 | 2.091 | 4.098 |
| 37 | C | -6.026 | 1.733 | 1.417 |
| 38 | O | -6.846 | 1.405 | 2.531 |
| 39 | C | -6.509 | 0.994 | 0.136 |
| 40 | C | -5.972 | 1.697 | -1.116 |
| 41 | C | -8.055 | 0.91 | 0.099 |
| 42 | C | -8.561 | 0.067 | -1.038 |
| 43 | C | -9.479 | 0.471 | -1.924 |
| 44 | C | -10.003 | -0.377 | -3.041 |
| 45 | N | -3.194 | 3.242 | 2.909 |
| 46 | C | -2.89 | 4.157 | 4.026 |
| 47 | C | -3.571 | 5.521 | 3.734 |
| 48 | O | -3.028 | 6.329 | 2.938 |
| 49 | C | -1.362 | 4.328 | 4.178 |
| 50 | C | -0.989 | 5.122 | 5.422 |
| 51 | N | -4.76 | 5.867 | 4.313 |
| 52 | C | -5.322 | 7.173 | 3.906 |
| 53 | C | -5.762 | 7.081 | 2.426 |
| 54 | O | -6.586 | 6.209 | 2.097 |
| 55 | C | -5.645 | 4.979 | 5.057 |
| 56 | N | -5.219 | 7.93 | 1.491 |

(continued)

| a | atom_type | $X_{a,1}$ | $X_{a,2}$ | $X_{a,3}$ |
|---|---|---|---|---|
| 57 | C | -4.241 | 8.964 | 1.814 |
| 58 | C | -5.612 | 7.747 | 0.073 |
| 59 | C | -6.315 | 9.01 | -0.487 |
| 60 | C | -7.163 | 8.742 | -1.752 |
| 61 | C | -8.397 | 7.883 | -1.446 |
| 62 | C | -7.581 | 10.072 | -2.394 |
| 63 | C | -4.4 | 7.312 | -0.795 |
| 64 | O | -4.305 | 7.644 | -1.989 |
| 65 | N | -3.453 | 6.514 | -0.203 |
| 66 | C | -2.335 | 5.993 | -0.996 |
| 67 | C | -2.32 | 4.452 | -0.908 |
| 68 | O | -2.799 | 3.869 | 0.1 |
| 69 | C | -0.958 | 6.62 | -0.556 |
| 70 | C | -1.013 | 8.148 | -0.659 |
| 71 | C | -0.537 | 6.164 | 0.844 |
| 72 | N | -1.782 | 3.725 | -1.934 |
| 73 | C | -1.252 | 4.328 | -3.154 |
| 74 | C | -1.767 | 2.236 | -1.837 |
| 75 | C | -2.514 | 1.591 | -3.032 |
| 76 | C | -2.441 | 0.046 | -3.049 |
| 77 | C | -3.144 | -0.59 | -1.844 |
| 78 | C | -3.018 | -0.499 | -4.361 |
| 79 | C | -0.298 | 1.752 | -1.777 |
| 80 | O | 0.472 | 1.873 | -2.758 |
| 81 | N | 0.133 | 1.189 | -0.612 |
| 82 | C | 1.538 | 0.756 | -0.453 |
| 83 | C | 1.77 | -0.664 | -1.063 |
| 84 | O | 2.248 | -1.602 | -0.401 |
| 85 | C | 1.948 | 0.812 | 1.017 |
| 86 | H | 1.718 | -2.791 | -5.128 |
| 87 | H | 0.464 | -1.592 | -4.821 |
| 88 | H | 2.155 | -1.093 | -4.934 |
| 89 | H | 2.669 | -2.437 | -2.86 |
| 90 | H | 1.087 | -0.008 | -2.908 |
| 91 | H | 1.467 | -7.114 | -2.222 |
| 92 | H | 0.902 | -6.404 | -3.732 |
| 93 | H | -0.905 | -8 | -1.846 |
| 94 | H | -2.34 | -8.273 | -3.858 |
| 95 | H | -1.221 | -7.311 | -4.815 |

(continued)

| a | atom_type | $X_{a,1}$ | $X_{a,2}$ | $X_{a,3}$ |
|---|---|---|---|---|
| 96 | H | -2.115 | -6.566 | -3.496 |
| 97 | H | -0.532 | -9.957 | -3.342 |
| 98 | H | 0.966 | -9.45 | -2.565 |
| 99 | H | 0.668 | -9.05 | -4.254 |
| 100 | H | -0.86 | -5.121 | -2.378 |
| 101 | H | -3.927 | -5.754 | 0.124 |
| 102 | H | -3.028 | -6.184 | -1.328 |
| 103 | H | -4.612 | -3.581 | -1.065 |
| 104 | H | -5.61 | -6.242 | -2.204 |
| 105 | H | -6.173 | -5.468 | -0.725 |
| 106 | H | -6.466 | -4.708 | -2.287 |
| 107 | H | -2.906 | -3.679 | -2.928 |
| 108 | H | -3.719 | -5.103 | -3.569 |
| 109 | H | -4.557 | -3.56 | -3.529 |
| 110 | H | -1.8 | -3.895 | -1.069 |
| 111 | H | -0.326 | -0.734 | 2.88 |
| 112 | H | -2.405 | -1.049 | 5.105 |
| 113 | H | -2.044 | 0.436 | 4.23 |
| 114 | H | -0.826 | -0.292 | 5.272 |
| 115 | H | -1.187 | -3.273 | 4.37 |
| 116 | H | 0.335 | -2.42 | 4.602 |
| 117 | H | -0.011 | -3.194 | 3.06 |
| 118 | H | -3.036 | -2.174 | 2.899 |
| 119 | H | -4.575 | 7.956 | 4.079 |
| 120 | H | -6.191 | 7.386 | 4.538 |
| 121 | H | -5.56 | 9.767 | -0.722 |
| 122 | H | -6.952 | 9.434 | 0.297 |
| 123 | H | -6.532 | 8.202 | -2.471 |
| 124 | H | -8.12 | 6.916 | -1.032 |
| 125 | H | -8.971 | 7.705 | -2.353 |
| 126 | H | -9.051 | 8.378 | -0.73 |
| 127 | H | -8.159 | 9.898 | -3.299 |
| 128 | H | -6.709 | 10.666 | -2.659 |
| 129 | H | -8.196 | 10.661 | -1.714 |
| 130 | H | -6.33 | 6.908 | 0.09 |
| 131 | H | -0.2 | 6.261 | -1.271 |
| 132 | H | -1.727 | 8.558 | 0.052 |
| 133 | H | -0.038 | 8.579 | -0.442 |
| 134 | H | -1.314 | 8.459 | -1.656 |

(continued)

| a | atom_type | $X_{a,1}$ | $X_{a,2}$ | $X_{a,3}$ |
|---|---|---|---|---|
| 135 | H | -0.408 | 5.084 | 0.874 |
| 136 | H | 0.408 | 6.625 | 1.123 |
| 137 | H | -1.284 | 6.436 | 1.588 |
| 138 | H | -2.541 | 6.31 | -2.032 |
| 139 | H | -3.519 | 6.228 | 0.777 |
| 140 | H | -2.078 | 1.966 | -3.962 |
| 141 | H | -3.559 | 1.918 | -3.018 |
| 142 | H | -1.379 | -0.241 | -3.008 |
| 143 | H | -3.003 | -1.668 | -1.863 |
| 144 | H | -4.213 | -0.39 | -1.864 |
| 145 | H | -2.745 | -0.213 | -0.904 |
| 146 | H | -2.911 | -1.58 | -4.399 |
| 147 | H | -4.076 | -0.26 | -4.454 |
| 148 | H | -2.501 | -0.075 | -5.219 |
| 149 | H | -2.275 | 1.994 | -0.89 |
| 150 | H | 2.963 | 0.445 | 1.136 |
| 151 | H | 1.897 | 1.831 | 1.395 |
| 152 | H | 1.293 | 0.191 | 1.625 |
| 153 | H | 2.152 | 1.464 | -1.04 |
| 154 | H | -0.521 | 1.036 | 0.167 |
| 155 | H | 2.45 | -5.44 | -0.987 |
| 156 | H | 3.099 | -4.804 | -2.514 |
| 157 | H | 2.816 | -3.712 | -1.132 |
| 158 | H | -0.596 | -6.672 | 1.821 |
| 159 | H | -1.111 | -5.074 | 2.408 |
| 160 | H | -2.326 | -6.221 | 1.814 |
| 161 | H | -0.824 | -2.08 | -0.569 |
| 162 | H | 0.154 | -2.934 | 0.64 |
| 163 | H | -0.229 | -1.221 | 0.854 |
| 164 | H | -4.91 | -0.921 | 3.723 |
| 165 | H | -6.086 | -0.554 | 2.457 |
| 166 | H | -4.946 | -1.891 | 2.226 |
| 167 | H | -3.891 | 1.824 | 0.914 |
| 168 | H | -6.101 | 2.831 | 1.233 |
| 169 | H | -6.369 | 1.656 | 3.353 |
| 170 | H | -6.114 | -0.035 | 0.151 |
| 171 | H | -6.381 | 2.702 | -1.199 |
| 172 | H | -4.887 | 1.781 | -1.093 |
| 173 | H | -6.251 | 1.148 | -2.012 |

(continued)

| a | atom_type | $X_{a,1}$ | $X_{a,2}$ | $X_{a,3}$ |
|---|---|---|---|---|
| 174 | H | -8.478 | 1.917 | 0.04 |
| 175 | H | -8.395 | 0.48 | 1.049 |
| 176 | H | -11.084 | -0.504 | -2.96 |
| 177 | H | -9.543 | -1.363 | -3.042 |
| 178 | H | -9.812 | 0.09 | -4.009 |
| 179 | H | -3.299 | 3.693 | 4.936 |
| 180 | H | -0.975 | 4.833 | 3.289 |
| 181 | H | -0.899 | 3.338 | 4.214 |
| 182 | H | 0.091 | 5.217 | 5.506 |
| 183 | H | -1.412 | 6.123 | 5.384 |
| 184 | H | -1.355 | 4.635 | 6.325 |
| 185 | H | -5.781 | 5.331 | 6.089 |
| 186 | H | -5.256 | 3.961 | 5.076 |
| 187 | H | -6.629 | 4.956 | 4.579 |
| 188 | H | -3.918 | 9.448 | 0.893 |
| 189 | H | -3.359 | 8.54 | 2.309 |
| 190 | H | -4.666 | 9.739 | 2.467 |
| 191 | H | -0.692 | 5.241 | -2.935 |
| 192 | H | -0.57 | 3.624 | -3.631 |
| 193 | H | -2.052 | 4.585 | -3.864 |
| 194 | H | -2.802 | 3.466 | 1.985 |
| 195 | H | -8.151 | -0.941 | -1.117 |
| 196 | H | -9.89 | 1.478 | -1.849 |

The coordinate data of atoms (X, Y, Z of each atom) of the main chain of cyclosporin A are shown below.

[Table 15]

| a | atom_type | $X_{a,1}$ | $X_{a,2}$ | $X_{a,3}$ |
|---|---|---|---|---|
| 1 | N | 1.43 | -0.813 | -2.378 |
| 2 | C | 1.642 | -2.092 | -3.076 |
| 4 | C | 0.611 | -3.168 | -2.61 |
| 6 | N | 1.053 | -4.355 | -2.093 |
| 8 | C | 0.084 | -5.48 | -1.944 |
| 13 | C | -0.076 | -5.848 | -0.44 |
| 15 | N | -1.132 | -5.359 | 0.299 |
| 17 | C | -2.213 | -4.49 | -0.237 |
| 22 | C | -2.674 | -3.519 | 0.893 |
| 24 | N | -1.838 | -2.487 | 1.246 |
| 26 | C | -2.251 | -1.613 | 2.374 |
| 30 | C | -2.805 | -0.261 | 1.849 |

(continued)

| a | atom_type | $X_{a,1}$ | $X_{a,2}$ | $X_{a,3}$ |
|---|---|---|---|---|
| 32 | N | -4.128 | 0.043 | 1.993 |
| 34 | C | -4.513 | 1.473 | 1.754 |
| 35 | C | -4.16 | 2.294 | 3.017 |
| 45 | N | -3.194 | 3.242 | 2.909 |
| 46 | C | -2.89 | 4.157 | 4.026 |
| 47 | C | -3.571 | 5.521 | 3.734 |
| 51 | N | -4.76 | 5.867 | 4.313 |
| 52 | C | -5.322 | 7.173 | 3.906 |
| 53 | C | -5.762 | 7.081 | 2.426 |
| 56 | N | -5.219 | 7.93 | 1.491 |
| 58 | C | -5.612 | 7.747 | 0.073 |
| 63 | C | -4.4 | 7.312 | -0.795 |
| 65 | N | -3.453 | 6.514 | -0.203 |
| 66 | C | -2.335 | 5.993 | -0.996 |
| 67 | C | -2.32 | 4.452 | -0.908 |
| 72 | N | -1.782 | 3.725 | -1.934 |
| 74 | C | -1.767 | 2.236 | -1.837 |
| 79 | C | -0.298 | 1.752 | -1.777 |
| 81 | N | 0.133 | 1.189 | -0.612 |
| 82 | C | 1.538 | 0.756 | -0.453 |
| 83 | C | 1.77 | -0.664 | -1.063 |

The values of all the components (3 x 3) of the inertia tensor for cyclosporin A are shown below.

[Table 16]

| | | | | | | |
|---|---|---|---|---|---|---|
| $I_{11}$ | $I_{21}$ | $I_{31}$ | | 751.9926 | 218.4299 | 113.1031 |
| $I_{12}$ | $I_{22}$ | $I_{32}$ | | 218.4299 | 317.4003 | -117.045 |
| $I_{13}$ | $I_{23}$ | $I_{33}$ | | 113.1031 | -117.045 | 773.4686 |

The values of all the components (3) of the principal moments of inertia for cyclosporin A are shown below.
$I_1$ = 187.0868, $I_2$ = 768.3052, $I_3$ = 887.4694
The values of a, b, and c for cyclosporin A are shown below.
a = 10.54819, b = 4.816724, c = 2.268403
The r value for cyclosporin A is shown below.
r value: 0.475683
An ellipsoid diagram for cyclosporin A is shown in Fig. 6.
The r values obtained in Example 2 are shown in the section of <Summary of results> which will be described later.

Example 3: r value is obtained from structure by MD calculation

Determination of the r value was carried out using cyclosporin A and isocyclosporin.
First, a two-dimensionally drawn structural formula of the cyclic peptide is input into Chem3D to create a three-dimensional structure. Fig. 7 shows three-dimensionally structured cyclosporin A and isocyclosporin.
Using the three-dimensional structures as initial structures, the structure optimization is carried out by a quantum

chemical calculation method (B3LYP/6-31G*, software: Gaussian) to obtain locally stable structures. In the locally stable structures, an electrostatic field for generating a cyclic peptide is obtained by a quantum chemical calculation method (B3LYP/6-31G*, software: Gaussian), and a point charge (RESP charge) is assigned to each atom so as to reproduce the electrostatic field. Next, the state of covalent bonds between the atoms is analyzed (Amber), and van der Waals parameters (gaff2) are assigned to each atom. These charges and van der Waals parameters are collectively referred to as a force field.

Fig. 8 shows structures of cyclosporin A and isocyclosporin after structure optimization by MD calculation using the three-dimensional structures created by Chem3D as initial structures.

Next, under the present force field, using the present locally stable structures as initial structures, molecular dynamics (MD) simulations are carried out in chloroform (software: Gromacs and plumed). As an efficient method for efficiently exploring a wide conformation space, the MD simulation employs a replica exchange MD method in which temperatures higher than room temperature are also used in addition to room temperature as temperatures at the time of the simulation. The temperatures used are six types (six types of replicas), and are as shown in the table below. The present temperature is applied only to the cyclic peptide and 298 K is always applied to chloroform present around the cyclic peptide.

[Table 17]

| | Temperature of CsA/chloroform [K] |
|---|---|
| Lineage 1 | 298/298 |
| Lineage 2 | 348/298 |
| Lineage 3 | 398/298 |
| Lineage 4 | 448/298 |
| Lineage 5 | 498/298 |
| Lineage 6 | 548/298 |

In a case where the calculation for 300 ns was carried out by the present replica exchange MD method, the most stable structure at room temperature was calculated. The structures of most stabilized cyclosporin A and isocyclosporin are shown in Fig. 9.

The main chain structure by MD calculation and the main chain structure by NMR + MD calculation are shown in Fig. 10. The main chain structure by MD calculation was a main chain structure (RMSD < 1 Å) similar to the structure determined conformation by carrying out NMR measurement in chloroform and MD calculation restrained by the NMR data.

The method described in Example 2 was applied to the present most stable structure to obtain the inertia tensor, the principal moments of inertia, a, b, and c, and then the r value.

The coordinate data (X, Y, Z of each atom) for the most stable structure of cyclosporin A are shown below.

[Table 18]

| a | atom_type | $X_{a,1}$ | $X_{a,2}$ | $X_{a,3}$ |
|---|---|---|---|---|
| 1 | C | -1.35 | -0.743 | 0.892 |
| 2 | C | -0.252 | -2.214 | 6.788 |
| 3 | C | -1.636 | -1.606 | 8.722 |
| 4 | C | -0.164 | -2.449 | 5.252 |
| 5 | O | 0.735 | -2.356 | 7.533 |
| 6 | C | -3.042 | -1.994 | 9.201 |
| 7 | C | -1.368 | -0.099 | 8.998 |
| 8 | H | -0.883 | -2.219 | 9.244 |
| 9 | C | 1.27 | -2.752 | 4.817 |
| 10 | H | -0.818 | -3.311 | 5.023 |
| 11 | N | -0.685 | -1.305 | 4.478 |

(continued)

| a | atom_type | $X_{a,1}$ | $X_{a,2}$ | $X_{a,3}$ |
|---|---|---|---|---|
| 12 | H | -3.153 | -1.8 | 10.266 |
| 13 | H | -3.8 | -1.422 | 8.671 |
| 14 | H | -3.229 | -3.051 | 9.026 |
| 15 | O | -1.603 | 0.75 | 8.108 |
| 16 | N | -0.897 | 0.298 | 10.223 |
| 17 | H | 1.69 | -3.545 | 5.43 |
| 18 | H | 1.3 | -3.059 | 3.774 |
| 19 | H | 1.893 | -1.867 | 4.928 |
| 20 | C | -2.011 | -1.216 | 4.162 |
| 21 | H | 0 | -0.634 | 4.11 |
| 22 | C | -0.758 | 1.761 | 10.483 |
| 23 | C | -0.429 | -0.627 | 11.249 |
| 24 | O | -2.847 | -1.996 | 4.668 |
| 25 | C | -2.399 | -0.197 | 3.06 |
| 26 | H | -1.269 | 2.26 | 9.649 |
| 27 | C | -1.435 | 2.18 | 11.809 |
| 28 | C | 0.753 | 2.131 | 10.526 |
| 29 | H | -1.262 | -1.086 | 11.804 |
| 30 | H | 0.169 | -1.433 | 10.813 |
| 31 | H | 0.2 | -0.084 | 11.954 |
| 32 | C | -3.784 | 0.459 | 3.265 |
| 33 | H | -1.617 | 0.573 | 2.971 |
| 34 | N | -2.369 | -0.957 | 1.77 |
| 35 | C | -2.92 | 1.768 | 11.923 |
| 36 | H | -1.351 | 3.268 | 11.9 |
| 37 | H | -0.872 | 1.759 | 12.647 |
| 38 | N | 1.377 | 2.673 | 9.42 |
| 39 | O | 1.402 | 1.896 | 11.563 |
| 40 | H | -4.514 | -0.313 | 3.524 |
| 41 | H | -4.098 | 0.889 | 2.309 |
| 42 | C | -3.816 | 1.564 | 4.345 |
| 43 | C | -3.4 | -1.972 | 1.553 |
| 44 | C | -3.788 | 2.411 | 10.833 |
| 45 | H | -2.979 | 0.674 | 11.799 |
| 46 | C | -3.455 | 2.111 | 13.32 |
| 47 | C | 2.759 | 3.13 | 9.574 |
| 48 | C | 0.678 | 3.049 | 8.161 |
| 49 | C | -5.132 | 2.349 | 4.244 |
| 50 | C | -3.64 | 0.994 | 5.756 |

(continued)

| a | atom_type | $X_{a,1}$ | $X_{a,2}$ | $X_{a,3}$ |
|---|---|---|---|---|
| 51 | H | -2.989 | 2.264 | 4.148 |
| 52 | H | -2.954 | -2.958 | 1.37 |
| 53 | H | -4.012 | -2.055 | 2.449 |
| 54 | H | -4.039 | -1.718 | 0.699 |
| 55 | O | -0.435 | 0.086 | 1.153 |
| 56 | C | -1.333 | -1.499 | -0.466 |
| 57 | H | -3.48 | 2.091 | 9.841 |
| 58 | H | -3.723 | 3.497 | 10.874 |
| 59 | H | -4.831 | 2.134 | 10.966 |
| 60 | H | -2.863 | 1.625 | 14.093 |
| 61 | H | -4.487 | 1.786 | 13.428 |
| 62 | H | -3.421 | 3.183 | 13.497 |
| 63 | H | 2.864 | 4.162 | 9.223 |
| 64 | H | 3.46 | 2.505 | 9.002 |
| 65 | H | 3.023 | 3.07 | 10.63 |
| 66 | H | -0.17 | 2.355 | 8.014 |
| 67 | C | 1.633 | 2.871 | 6.951 |
| 68 | C | 0.15 | 4.509 | 8.246 |
| 69 | H | -5.247 | 2.793 | 3.257 |
| 70 | H | -5.158 | 3.151 | 4.979 |
| 71 | H | -5.988 | 1.702 | 4.424 |
| 72 | H | -4.372 | 0.214 | 5.953 |
| 73 | H | -3.77 | 1.774 | 6.501 |
| 74 | H | -2.649 | 0.571 | 5.895 |
| 75 | C | 0.025 | -2.229 | -0.674 |
| 76 | H | -2.156 | -2.228 | -0.517 |
| 77 | N | -1.573 | -0.523 | -1.544 |
| 78 | O | 1.85 | 3.822 | 6.157 |
| 79 | N | 2.264 | 1.672 | 6.756 |
| 80 | H | -0.202 | 4.696 | 9.267 |
| 81 | C | -0.982 | 4.831 | 7.242 |
| 82 | H | 0.987 | 5.191 | 8.07 |
| 83 | H | 0.814 | -1.464 | -0.642 |
| 84 | C | 0.08 | -2.919 | -2.042 |
| 85 | C | 0.277 | -3.229 | 0.461 |
| 86 | H | -0.766 | 0.01 | -1.882 |
| 87 | C | -2.849 | -0.252 | -1.96 |
| 88 | C | 3.214 | 1.622 | 5.601 |
| 89 | C | 1.866 | 0.446 | 7.435 |

(continued)

| a | atom_type | $X_{a,1}$ | $X_{a,2}$ | $X_{a,3}$ |
|---|---|---|---|---|
| 90 | H | -0.668 | 4.482 | 6.25 |
| 91 | C | -1.194 | 6.349 | 7.162 |
| 92 | C | -2.292 | 4.123 | 7.61 |
| 93 | H | -0.718 | -3.652 | -2.145 |
| 94 | H | -0.017 | -2.191 | -2.843 |
| 95 | H | 1.029 | -3.434 | -2.168 |
| 96 | H | 0.298 | -2.727 | 1.427 |
| 97 | H | 1.233 | -3.73 | 0.322 |
| 98 | H | -0.498 | -3.993 | 0.492 |
| 99 | O | -3.825 | -0.88 | -1.514 |
| 100 | C | -3.044 | 0.908 | -2.982 |
| 101 | H | 3.772 | 2.571 | 5.638 |
| 102 | C | 4.239 | 0.454 | 5.684 |
| 103 | C | 2.353 | 1.579 | 4.308 |
| 104 | H | 1.901 | -0.396 | 6.745 |
| 105 | H | 2.513 | 0.213 | 8.292 |
| 106 | H | 0.841 | 0.528 | 7.796 |
| 107 | H | -0.28 | 6.852 | 6.853 |
| 108 | H | -1.972 | 6.589 | 6.441 |
| 109 | H | -1.494 | 6.756 | 8.126 |
| 110 | H | -3.065 | 4.36 | 6.882 |
| 111 | H | -2.648 | 4.442 | 8.588 |
| 112 | H | -2.172 | 3.042 | 7.628 |
| 113 | H | -3.21 | 1.824 | -2.386 |
| 114 | C | -4.284 | 0.659 | -3.877 |
| 115 | N | -1.799 | 1.166 | -3.745 |
| 116 | C | 5.053 | 0.364 | 4.386 |
| 117 | H | 3.688 | -0.491 | 5.791 |
| 118 | C | 5.173 | 0.622 | 6.889 |
| 119 | N | 2.474 | 2.587 | 3.393 |
| 120 | O | 1.524 | 0.649 | 4.148 |
| 121 | H | -4.933 | -0.044 | -3.345 |
| 122 | H | -3.98 | 0.165 | -4.806 |
| 123 | C | -5.097 | 1.932 | -4.208 |
| 124 | C | -1.1 | 2.318 | -3.473 |
| 125 | C | -1.303 | 0.125 | -4.643 |
| 126 | H | 5.76 | -0.46 | 4.441 |
| 127 | H | 5.619 | 1.277 | 4.209 |
| 128 | H | 4.405 | 0.195 | 3.528 |

(continued)

| a | atom_type | $X_{a,1}$ | $X_{a,2}$ | $X_{a,3}$ |
|---|---|---|---|---|
| 129 | H | 5.768 | 1.528 | 6.795 |
| 130 | H | 4.616 | 0.683 | 7.821 |
| 131 | H | 5.857 | -0.221 | 6.96 |
| 132 | C | 3.494 | 3.631 | 3.484 |
| 133 | C | 1.427 | 2.737 | 2.342 |
| 134 | C | -6.35 | 1.559 | 5.011 |
| 135 | H | -5.427 | 2.372 | -3.253 |
| 136 | C | -4.266 | 2.988 | -4.947 |
| 137 | O | 1.58 | 3.199 | 2.732 |
| 138 | C | 0.324 | 2.522 | -4.037 |
| 139 | H | -0.32 | -0.246 | -4.33 |
| 140 | H | -1.223 | 0.49 | -5.676 |
| 141 | H | -2.001 | -0.711 | -4.639 |
| 142 | H | 3.279 | 4.347 | 4.289 |
| 143 | H | 4.482 | 3.194 | 3.667 |
| 144 | H | 3.538 | 4.162 | 2.533 |
| 145 | H | 0.753 | 1.876 | 2.467 |
| 146 | C | 2.093 | 2.698 | 0.95 |
| 147 | C | 0.648 | 4.089 | 2.587 |
| 148 | H | -6.957 | 0.835 | -4.472 |
| 149 | H | -6.082 | 1.123 | -5.972 |
| 150 | H | -6.961 | 2.438 | -5.203 |
| 151 | H | -3.447 | 3.344 | -4.327 |
| 152 | H | -3.851 | 2.585 | -5.869 |
| 153 | H | -4.887 | 3.843 | -5.207 |
| 154 | H | 0.637 | 1.711 | -4.704 |
| 155 | H | 0.371 | 3.464 | -4.594 |
| 156 | N | 1.278 | 2.606 | -2.908 |
| 157 | N | 1.882 | 1.584 | 0.187 |
| 158 | O | 2.822 | 3.631 | 0.547 |
| 159 | O | 0.058 | 4.062 | 3.877 |
| 160 | H | 1.397 | 4.913 | 2.543 |
| 161 | C | -0.431 | 4.372 | 1.512 |
| 162 | C | 1.573 | 3.945 | -2.409 |
| 163 | C | 1.582 | 1.438 | -2.268 |
| 164 | H | 1.211 | 0.864 | 0.49 |
| 165 | C | 2.59 | 1.405 | -1.093 |
| 166 | H | 0.746 | 4.017 | 4.573 |
| 167 | C | -1.59 | 3.346 | 1.549 |

(continued)

| a | atom_type | $X_{a,1}$ | $X_{a,2}$ | $X_{a,3}$ |
|---|---|---|---|---|
| 168 | H | 0.062 | 4.297 | 0.528 |
| 169 | C | -0.96 | 5.803 | 1.673 |
| 170 | H | 0.639 | 4.462 | -2.161 |
| 171 | H | 2.194 | 3.905 | -1.514 |
| 172 | H | 2.089 | 4.536 | -3.177 |
| 173 | O | 1.051 | 0.367 | -2.667 |
| 174 | H | 3.299 | 2.244 | -1.168 |
| 175 | C | 3.379 | 0.072 | -1.112 |
| 176 | H | -2.109 | 3.42 | 2.508 |
| 177 | C | -2.555 | 3.534 | 0.409 |
| 178 | H | -1.168 | 2.332 | 1.491 |
| 179 | H | -0.154 | 6.529 | 1.568 |
| 180 | H | -1.715 | 6.02 | 0.921 |
| 181 | H | -1.407 | 5.935 | 2.655 |
| 182 | H | 3.866 | -0.027 | -2.086 |
| 183 | H | 2.674 | -0.76 | -1.032 |
| 184 | C | 4.417 | -0.01 | -0.003 |
| 185 | C | -3.877 | 3.682 | 0.563 |
| 186 | H | -2.132 | 3.537 | -0.595 |
| 187 | H | 5.141 | 0.798 | -0.083 |
| 188 | H | 3.948 | 0.058 | 0.977 |
| 189 | H | 4.957 | -0.953 | -0.052 |
| 190 | H | -4.31 | 3.685 | 1.564 |
| 191 | C | -4.835 | 3.851 | -0.576 |
| 192 | H | -5.573 | 3.046 | -0.591 |
| 193 | H | -4.314 | 3.855 | -1.531 |
| 194 | H | -5.39 | 4.786 | -0.485 |
| 195 | N | -1.483 | -1.893 | 7.291 |
| 196 | H | -2.271 | -1.739 | 6.657 |

The coordinate data of atoms (X, Y, Z of each atom) of the main chain of cyclosporin A are shown below.

[Table 19]

| a | atom_type | $X_{a,1}$ | $X_{a,2}$ | $X_{a,3}$ |
|---|---|---|---|---|
| 1 | C | -1.35 | -0.743 | 0.892 |
| 56 | C | -1.333 | -1.499 | -0.466 |
| 77 | N | -1.573 | -0.523 | -1.544 |
| 87 | C | -2.849 | -0.252 | -1.96 |
| 100 | C | -3.044 | 0.908 | -2.982 |
| 115 | N | -1.799 | 1.166 | -3.745 |

(continued)

| a | atom_type | $X_{a,1}$ | $X_{a,2}$ | $X_{a,3}$ |
|---|---|---|---|---|
| 124 | C | -1.1 | 2.318 | -3.473 |
| 138 | C | 0.324 | 2.522 | -4.037 |
| 156 | N | 1.278 | 2.606 | -2.908 |
| 163 | C | 1.582 | 1.438 | -2.268 |
| 165 | C | 2.59 | 1.405 | -1.093 |
| 157 | N | 1.882 | 1.584 | 0.187 |
| 146 | C | 2.093 | 2.698 | 0.95 |
| 133 | C | 1.427 | 2.737 | 2.342 |
| 119 | N | 2.474 | 2.587 | 3.393 |
| 103 | C | 2.353 | 1.579 | 4.308 |
| 88 | C | 3.214 | 1.622 | 5.601 |
| 79 | N | 2.264 | 1.672 | 6.756 |
| 67 | C | 1.633 | 2.871 | 6.951 |
| 48 | C | 0.678 | 3.049 | 8.161 |
| 38 | N | 1.377 | 2.673 | 9.42 |
| 28 | C | 0.753 | 2.131 | 10.526 |
| 22 | C | -0.758 | 1.761 | 10.483 |
| 16 | N | -0.897 | 0.298 | 10.223 |
| 7 | C | -1.368 | -0.099 | 8.998 |
| 3 | C | -1.636 | -1.606 | 8.722 |
| 195 | N | -1.483 | -1.893 | 7.291 |
| 2 | C | -0.252 | -2.214 | 6.788 |
| 4 | C | -0.164 | -2.449 | 5.252 |
| 11 | N | -0.685 | -1.305 | 4.478 |
| 20 | C | -2.011 | -1.216 | 4.162 |
| 25 | C | -2.399 | -0.197 | 3.06 |
| 34 | N | -2.369 | -0.957 | 1.77 |

The values of all the components (3 x 3) of the inertia tensor for cyclosporin A are shown below.

[Table 20]

| | | | | | |
|---|---|---|---|---|---|
| $I_{11}$ | $I_{21}$ | $I_{31}$ | 802.3409 | -62.7456 | -34.5588 |
| $I_{12}$ | $I_{22}$ | $I_{32}$ | -62.7456 | 812.9554 | 26.37697 |
| $I_{13}$ | $I_{23}$ | $I_{33}$ | -34.5588 | 26.37697 | 200.5268 |

The values of all the components (3) of the principal moments of inertia for cyclosporin A are shown below.
$I_1$ = 197.6983, $I_2$ = 744.7818, $I_3$ = 873.343

The values of a, b, and c for cyclosporin A are shown below.
a = 10.37343, b = 4.971582, c = 2.288593

The r value for cyclosporin A is shown below.
r value: 0.494714

An ellipsoid diagram for cyclosporin A is shown in Fig. 11.

The coordinate data (X, Y, Z of each atom) for the most stable structure of isocyclosporin are shown below.

[Table 21]

| a | atom_type | Xa,1 | Xa,2 | Xa,3 |
|---|---|---|---|---|
| 1 | C | 0.598 | -0.424 | 2.132 |
| 2 | N | 0.104 | -0.766 | 0.803 |
| 3 | C | 0.19 | -2.017 | 0.246 |
| 4 | C | 0.835 | -3.194 | 1.038 |
| 5 | C | 0.651 | -4.51 | 0.242 |
| 6 | C | 1.324 | -5.743 | 0.881 |
| 7 | C | 0.914 | -7.016 | 0.128 |
| 8 | C | 2.851 | -5.607 | 0.931 |
| 9 | N | 0.338 | -3.326 | 2.431 |
| 10 | C | -1.083 | -3.612 | 2.619 |
| 11 | C | 1.241 | -3.255 | 3.456 |
| 12 | C | 0.798 | -3.483 | 4.93 |
| 13 | C | 1.701 | -4.532 | 5.586 |
| 14 | N | 0.884 | -2.215 | 5.684 |
| 15 | C | -0.133 | -1.301 | 5.609 |
| 16 | C | -0.032 | 0.015 | 6.416 |
| 17 | C | -0.201 | 1.227 | 5.478 |
| 18 | N | 1.202 | 0.109 | 7.201 |
| 19 | C | 1.186 | 0.412 | 8.542 |
| 20 | C | 2.608 | 0.526 | 9.185 |
| 21 | C | 2.529 | 0.761 | 10.718 |
| 22 | C | 2.517 | -0.531 | 11.572 |
| 23 | C | 1.365 | -1.469 | 11.197 |
| 24 | C | 3.864 | -1.266 | 11.534 |
| 25 | N | 3.337 | 1.618 | 8.493 |
| 26 | C | 2.625 | 2.891 | 8.371 |
| 27 | C | 4.575 | 1.509 | 7.903 |
| 28 | C | 5.475 | 0.255 | 8.111 |
| 29 | C | 6.823 | 0.683 | 8.769 |
| 30 | C | 7.744 | -0.525 | 8.978 |
| 31 | C | 6.578 | 1.419 | 10.091 |
| 32 | N | 5.749 | -0.368 | 6.803 |
| 33 | C | 4.794 | -1.04 | 6.117 |
| 34 | O | 3.643 | -1.22 | 6.607 |
| 35 | O | 5.017 | 2.444 | 7.203 |
| 36 | O | 0.141 | 0.585 | 9.176 |
| 37 | O | -1.146 | -1.525 | 4.916 |
| 38 | O | 2.446 | -2.974 | 3.223 |

(continued)

| a | atom_type | Xa,1 | Xa,2 | Xa,3 |
|---|---|---|---|---|
| 39 | O | -0.184 | -2.207 | -0.931 |
| 40 | C | -0.358 | 0.363 | -0.061 |
| 41 | C | 1.436 | 1.213 | 0.648 |
| 42 | C | -2.315 | 2.024 | -0.332 |
| 43 | C | -3.486 | 2.668 | 0.422 |
| 44 | C | -1.511 | 3.086 | -1.093 |
| 45 | C | 0.911 | 1.192 | 0.395 |
| 46 | N | 1.632 | 0.92 | -1.528 |
| 47 | C | 1.04 | 0.212 | -2.665 |
| 48 | C | 2.877 | 1.724 | -1.699 |
| 49 | C | 3.965 | 1.034 | -2.58 |
| 50 | C | 4.292 | -0.364 | -2.038 |
| 51 | C | 5.23 | 1.9 | -2.617 |
| 52 | C | 2.484 | 3.132 | -2.187 |
| 53 | O | 3.295 | 4.099 | -1.699 |
| 54 | C | 2.893 | 5.493 | -1.872 |
| 55 | C | 4.128 | 6.328 | -2.288 |
| 56 | C | 5.324 | 6.135 | -1.349 |
| 57 | C | 4.511 | 6.001 | -3.755 |
| 58 | C | 5.575 | 6.913 | -4.302 |
| 59 | C | 6.704 | 6.487 | -4.883 |
| 60 | C | 7.753 | 7.392 | -5.445 |
| 61 | C | 2.181 | 5.958 | -0.551 |
| 62 | N | 1.907 | 7.408 | -0.602 |
| 63 | C | 0.517 | 7.766 | -0.858 |
| 64 | C | 2.978 | 5.653 | 0.741 |
| 65 | N | 3.111 | 4.341 | 1.104 |
| 66 | C | 3.791 | 3.959 | 2.342 |
| 67 | C | 2.758 | 3.472 | 3.406 |
| 68 | C | 1.852 | 4.597 | 3.887 |
| 69 | C | 4.811 | 2.838 | 2.04 |
| 70 | N | 5.763 | 2.515 | 2.979 |
| 71 | C | 5.984 | 3.236 | 4.23 |
| 72 | C | 6.673 | 1.387 | 2.719 |
| 73 | C | 6.498 | 0.254 | 3.755 |
| 74 | N | 5.387 | -0.546 | 3.716 |
| 75 | C | 4.294 | -0.324 | 2.773 |
| 76 | C | 5.154 | -1.628 | 4.731 |
| 77 | C | 6.343 | -2.619 | 4.832 |

(continued)

| a | atom_type | Xa,1 | Xa,2 | Xa,3 |
|---|---|---|---|---|
| 78 | C | 6.591 | -3.415 | 3.529 |
| 79 | C | 7.977 | -4.072 | 3.566 |
| 80 | C | 5.497 | -4.462 | 3.284 |
| 81 | O | 7.386 | 0.096 | 4.627 |
| 82 | O | 4.746 | 2.186 | 0.972 |
| 83 | O | 3.447 | 6.574 | 1.438 |
| 84 | O | 1.503 | 3.373 | -2.887 |
| 85 | O | 1.341 | 2.021 | 0.446 |
| 86 | H | -0.139 | -0.652 | 2.915 |
| 87 | H | 1.523 | -0.962 | 2.356 |
| 88 | H | 0.817 | 0.644 | 2.161 |
| 89 | H | 1.913 | -2.974 | 1.137 |
| 90 | H | -0.42 | -4.705 | 0.125 |
| 91 | H | 1.043 | -4.352 | -0.765 |
| 92 | H | 0.959 | -5.834 | 1.918 |
| 93 | H | 1.249 | -6.981 | -0.906 |
| 94 | H | 1.353 | -7.897 | 0.592 |
| 95 | H | 0.167 | -7.137 | 0.126 |
| 96 | H | 3.147 | -4.756 | 1.539 |
| 97 | H | 3.262 | -5.476 | -0.067 |
| 98 | H | 3.298 | -6.5 | 1.362 |
| 99 | H | -1.259 | -4.669 | 2.867 |
| 100 | H | -1.505 | -2.987 | 3.413 |
| 101 | H | -1.612 | -3.397 | 1.691 |
| 102 | H | -0.252 | -3.814 | 4.971 |
| 103 | H | 1.599 | -5.492 | 5.085 |
| 104 | H | 2.741 | -4.223 | 5.522 |
| 105 | H | 1.441 | -4.663 | 6.635 |
| 106 | H | 1.787 | -1.984 | 6.111 |
| 107 | H | -0.858 | 0.023 | 7.147 |
| 108 | H | 0.648 | 1.322 | 4.802 |
| 109 | H | -0.279 | 2.146 | 6.054 |
| 110 | H | -1.102 | 1.109 | 4.883 |
| 111 | H | 2.105 | 0.006 | 6.732 |
| 112 | H | 3.13 | -0.419 | 8.972 |
| 113 | H | 1.617 | 1.326 | 10.93 |
| 114 | H | 3.376 | 1.382 | 11.029 |
| 115 | H | 2.355 | -0.205 | 12.611 |
| 116 | H | 1.296 | -2.289 | 11.909 |

(continued)

| a | atom_type | Xa,1 | Xa,2 | Xa,3 |
|---|---|---|---|---|
| 117 | H | 0.419 | -0.934 | 11.19 |
| 118 | H | 1.514 | -1.901 | 10.209 |
| 119 | H | 3.852 | -2.117 | 12.21 |
| 120 | H | 4.678 | -0.608 | 11.835 |
| 121 | H | 4.088 | -1.642 | 10.536 |
| 122 | H | 1.784 | 2.819 | 7.668 |
| 123 | H | 2.224 | 3.196 | 9.342 |
| 124 | H | 3.324 | 3.643 | 8.007 |
| 125 | H | 4.986 | -0.499 | 8.749 |
| 126 | H | 7.312 | 1.381 | 8.074 |
| 127 | H | 7.292 | -1.249 | 9.653 |
| 128 | H | 8.689 | -0.209 | 9.41 |
| 129 | H | 7.956 | -1.028 | 8.037 |
| 130 | H | 5.985 | 2.317 | 9.933 |
| 131 | H | 6.053 | 0.785 | 10.805 |
| 132 | H | 7.523 | 1.715 | 10.54 |
| 133 | H | 6.624 | -0.125 | 6.318 |
| 134 | H | -0.786 | -0.104 | -0.96 |
| 135 | H | -2.08 | 0.547 | 1.231 |
| 136 | H | -0.951 | 1.893 | 1.354 |
| 137 | H | -2.736 | 1.32 | -1.069 |
| 138 | H | -3.127 | 3.372 | 1.17 |
| 139 | H | -4.134 | 3.209 | -0.263 |
| 140 | H | -4.083 | 1.914 | 0.93 |
| 141 | H | -2.167 | 3.67 | -1.735 |
| 142 | H | -0.744 | 2.639 | -1.721 |
| 143 | H | -1.021 | 3.77 | -0.403 |
| 144 | H | 1.783 | 0.083 | -3.448 |
| 145 | H | 0.679 | -0.779 | -2.368 |
| 146 | H | 0.202 | 0.777 | -3.095 |
| 147 | H | 3.307 | 1.836 | -0.686 |
| 148 | H | 3.59 | 0.935 | -3.611 |
| 149 | H | 4.682 | -0.297 | -1.025 |
| 150 | H | 5.045 | -0.844 | -2.659 |
| 151 | H | 3.411 | -1.001 | -2.021 |
| 152 | H | 5.592 | 2.092 | -1.61 |
| 153 | H | 6.017 | 1.395 | -3.173 |
| 154 | H | 5.04 | 2.857 | -3.098 |
| 155 | H | 2.147 | 5.517 | -2.688 |

(continued)

| a | atom_type | Xa,1 | Xa,2 | Xa,3 |
|---|---|---|---|---|
| 156 | H | 3.82 | 7.385 | -2.246 |
| 157 | H | 6.187 | 6.678 | -1.728 |
| 158 | H | 5.102 | 6.505 | -0.351 |
| 159 | H | 5.593 | 5.084 | -1.275 |
| 160 | H | 4.836 | 4.958 | -3.828 |
| 161 | H | 3.608 | 6.09 | -4.376 |
| 162 | H | 5.387 | 7.983 | -4.218 |
| 163 | H | 6.894 | 5.416 | -4.963 |
| 164 | H | 8.715 | 7.227 | -4.956 |
| 165 | H | 7.904 | 7.201 | 6.509 |
| 166 | H | 7.485 | 8.439 | -5.323 |
| 167 | H | 1.232 | 5.386 | -0.49 |
| 168 | H | 2.248 | 7.826 | 0.274 |
| 169 | H | 0.207 | 7.418 | -1.848 |
| 170 | H | -0.189 | 7.339 | -0.122 |
| 171 | H | 0.413 | 8.853 | -0.834 |
| 172 | H | 2.633 | 3.588 | 0.6 |
| 173 | H | 4.296 | 4.868 | 2.708 |
| 174 | H | 3.291 | 3.036 | 4.258 |
| 175 | H | 2.157 | 2.673 | 2.963 |
| 176 | H | 1.309 | 5.036 | 3.054 |
| 177 | H | 2.43 | 5.387 | 4.361 |
| 178 | H | 1.127 | 4.225 | 4.607 |
| 179 | H | 5.547 | 2.721 | 5.096 |
| 180 | H | 7.06 | 3.328 | 4.409 |
| 181 | H | 5.564 | 4.24 | 4.177 |
| 182 | H | 6.489 | 1.047 | 1.693 |
| 183 | H | 7.715 | 1.734 | 2.788 |
| 184 | H | 3.781 | -1.268 | 2.58 |
| 185 | H | 3.554 | 0.384 | 3.175 |
| 186 | H | 4.659 | 0.083 | 1.829 |
| 187 | H | 4.265 | -2.177 | 4.387 |
| 188 | H | 7.25 | -2.072 | 5.099 |
| 189 | H | 6.135 | -3.314 | 5.653 |
| 190 | H | 6.578 | -2.7 | 2.691 |
| 191 | H | 8.051 | -4.78 | 4.389 |
| 192 | H | 8.171 | -4.613 | 2.642 |
| 193 | H | 8.757 | -3.325 | 3.693 |
| 194 | H | 5.484 | -5.199 | 4.085 |

(continued)

| a | atom_type | Xa,1 | Xa,2 | Xa,3 |
|---|---|---|---|---|
| 195 | H | 5.681 | -4.99 | 2.351 |
| 196 | H | 4.512 | -4.003 | 3.228 |

The coordinate data of atoms (X, Y, Z of each atom) of the main chain of isocyclosporin are shown below.

[Table 22]

| a | atom type | $X_{a,1}$ | $X_{a,2}$ | Xa,3 |
|---|---|---|---|---|
| 2 | N | 0.104 | -0.766 | 0.803 |
| 40 | C | -0.358 | 0.363 | -0.061 |
| 45 | C | 0.911 | 1.192 | -0.395 |
| 46 | N | 1.632 | 0.92 | -1.528 |
| 48 | C | 2.877 | 1.724 | -1.699 |
| 52 | C | 2.484 | 3.132 | -2.187 |
| 53 | O | 3.295 | 4.099 | -1.699 |
| 54 | C | 2.893 | 5.493 | -1.872 |
| 61 | C | 2.181 | 5.958 | -0.551 |
| 64 | C | 2.978 | 5.653 | 0.741 |
| 65 | N | 3.111 | 4.341 | 1.104 |
| 66 | C | 3.791 | 3.959 | 2.342 |
| 69 | C | 4.811 | 2.838 | 2.04 |
| 70 | N | 5.763 | 2.515 | 2.979 |
| 72 | C | 6.673 | 1.387 | 2.719 |
| 73 | C | 6.498 | 0.254 | 3.755 |
| 74 | N | 5.387 | -0.546 | 3.716 |
| 76 | C | 5.154 | -1.628 | 4.731 |
| 33 | C | 4.794 | -1.04 | 6.117 |
| 32 | N | 5.749 | -0.368 | 6.803 |
| 28 | C | 5.475 | 0.255 | 8.111 |
| 27 | C | 4.575 | 1.509 | 7.903 |
| 25 | N | 3.337 | 1.618 | 8.493 |
| 20 | C | 2.608 | 0.526 | 9.185 |
| 19 | C | 1.186 | 0.412 | 8.542 |
| 18 | N | 1.202 | 0.109 | 7.201 |
| 16 | C | -0.032 | 0.015 | 6.416 |
| 15 | C | -0.133 | -1.301 | 5.609 |
| 14 | N | 0.884 | -2.215 | 5.684 |
| 12 | C | 0.798 | -3.483 | 4.93 |
| 11 | C | 1.241 | -3.255 | 3.456 |
| 9 | N | 0.338 | -3.326 | 2.431 |
| 4 | C | 0.835 | -3.194 | 1.038 |

(continued)

| a | atom type | $X_{a,1}$ | $X_{a,2}$ | Xa,3 |
|---|---|---|---|---|
| 3 | C | 0.19 | -2.017 | 0.246 |

The values of all the components (3 x 3) of the inertia tensor for isocyclosporin are shown below.

[Table 23]

| | | | | | | |
|---|---|---|---|---|---|---|
| $I_{11}$ | $I_{21}$ | $I_{31}$ | | 626.3276 | -57.4717 | -43.5872 |
| $I_{12}$ | $I_{22}$ | $I_{32}$ | | -57.4717 | 549.1581 | 117.5674 |
| $I_{13}$ | $I_{23}$ | $I_{33}$ | | -43.5872 | 117.5674 | 376.3207 |

The values of all the components (3) of the principal moments of inertia for isocyclosporin are shown below.
$I_1$ = 316.2555, $I_2$ = 546.2999, $I_3$ = 689.2509
The values of a, b, and c for isocyclosporin are shown below.
a = 8.221633, b = 5.810782, c = 3.569731
The r value for isocyclosporin is shown below.
r value: 0.716695
An ellipsoid diagram for isocyclosporin is shown in Fig. 12.

Example 4: Evaluation of cell membrane permeability

<Preparation>

300 μL of MDCK II cells (ECACC standard cell line) at a density of $1.0 \times 10^6$ cells/mL were seeded in an insert (dedicated for a 24-well plate, pore diameter: 3.0 μm, manufactured by Corning Incorporated), and cultured at 37°C in a 5% $CO_2$ environment. After 3 days, the electric resistance value of the cell layer (measuring device: Millicell (registered trademark) ERS-2 (manufactured by Millipore Corporation), electrode: ENDOHM-6 (manufactured by WPI, Inc.)) was measured, and it was confirmed that the cell layer had high barrier properties (> 100 $\Omega \cdot cm^2$).

<Permeation test>

The insert was washed by being immersed in a Hank's Balanced Salt Solution (HBSS) (phenol red-free), 200 μL of a sample prepared at 10 μmol/L/HBSS was added thereto, and the insert was allowed to stand in a low-adsorption 24-well plate containing 900 μL of HBSS (37°C, 5% $CO_2$). After 2 hours, each liquid of the upper layer (apical) and the lower layer (basal) of the insert (10 μL for apical and 500 μL for basal) was recovered. After testing, no leakage was confirmed with Lucifer Yellow, which is a non-permeable fluorescent dye.

<Quantification>

The device used was LC/MS/MS (triple quadrupole type).

Eluent:

A) 5 mmol/L ammonium formate, 0.2% formic acid/$H_2O$
B) 0.1% formic acid/MeCN

Flow rate: 0.5 mL/min
Injection volume: 2 μL
Column: ACQUITY UPLC BEH C18 Column, 1.7 μm, 2.1 mm × 50 mm (manufactured by Waters Corporation)
Temperature: 70°C
Gradient (%B): 2% (0 to 0.5 min) → 98% (2 to 3 min) → 2% (3 to 5 min) Ionization: ESI
Detection mode: MRM (positive)

Based on the calculation expression shown in the following expression, a permeability coefficient $P_{app}$, which represents the membrane permeability, was calculated from each quantitative value.

$$P_{app} = V/C0 \times 1/S \times dC/dt$$

V: basal volume (0.9 mL)
C0: initial concentration (10 μM)
S: Surface area of single layer membrane (0.33 $cm^2$)
dC/dt: concentration change at basal [μM/s]

<Summary of results>

[Table 24]

| | Molecular shape factor (r) | | Cell membrane permeability $P_{app}$ x $10^{-6}$ cm/s |
|---|---|---|---|
| | Using NMR data | Calculation | |
| Compound 1 | 0.53 | None | 1.1 |
| Compound 2 | 0.79 | None | 0.04 |
| Cyclosporin A | 0.48 | 0.49 | 2.1 |
| Isocyclosporin | None | 0.72 | 0.2 |

The cyclic peptide with a molecular shape factor (r) in a range of 0.4 to 0.6 was found to have high cell membrane permeability.

## Claims

1. A method for predicting cell membrane permeability of a cyclic peptide, the method comprising:

   a first step of acquiring a structure of the cyclic peptide;
   a second step of calculating a molecular shape factor r which is calculated by Expression (1) after a step of carrying out an ellipsoidal approximation for obtaining each of axis lengths a, b, and c in a case where an axis length in a longest axis direction of a main chain structure is denoted by a, and axis lengths in two other directions which are orthogonal to a and are orthogonal to each other are denoted by b and c in the structure acquired in the first step; and

$$r = \frac{2\sqrt{b^2 + c^2}}{\sqrt{a^2 + b^2} + \sqrt{c^2 + a^2}} \qquad (1)$$

   a third step of determining that the cyclic peptide having the molecular shape factor r in a range of 0.4 to 0.6 has cell membrane permeability.

2. The method according to claim 1,
wherein, in the first step, the structure of the cyclic peptide is acquired by X-ray crystallography.

3. The method according to claim 1,
wherein, in the first step, the structure of the cyclic peptide is acquired by molecular dynamics calculation.

4. The method according to claim 1,
wherein, in the first step, the structure of the cyclic peptide is acquired by acquiring positional structural information of the cyclic peptide by two-dimensional $^1$H-NMR measurement and then carrying out structuring by computational chemistry based on the acquired positional structural information.

5. The method according to claim 4,

wherein the two-dimensional $^1$H-NMR measurement is a measurement by at least one of nuclear Overhauser effect spectroscopy, also referred to as NOESY, or rotating frame nuclear Overhauser effect spectroscopy, also referred to as ROESY.

**6.** The method according to claim 4,
wherein the two-dimensional $^1$H-NMR measurement is carried out at a temperature of 20°C to 60°C.

**7.** The method according to claim 4,
wherein the two-dimensional $^1$H-NMR measurement is carried out in dimethyl sulfoxide, dimethylformamide, dimethylacetamide, dichloromethane, chloroform, water, methanol, ethanol, propanol, tetrahydrofuran, or acetonitrile.

**8.** The method according to claim 4,
wherein the computational chemistry is a molecular dynamics method.

**9.** The method according to any one of claims 1 to 8,
wherein the cyclic peptide is non-ionic in a physiological environment.

**10.** The method according to any one of claims 1 to 8,
wherein the main chain structure of the cyclic peptide contains a sulfur atom.

# FIG. 1

COMPOUND 1

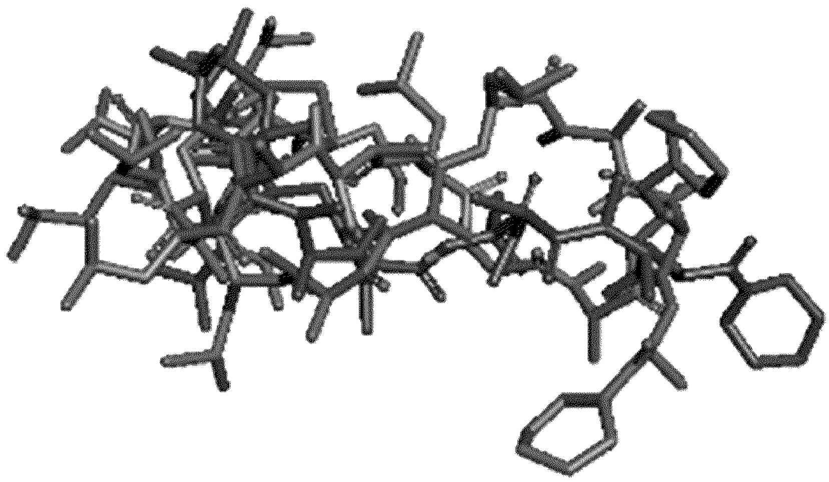

COMPOUND 2

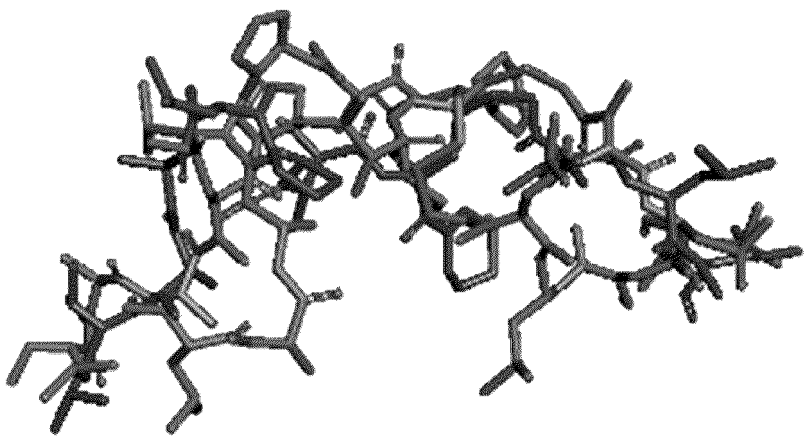

CYCLOSPORIN A

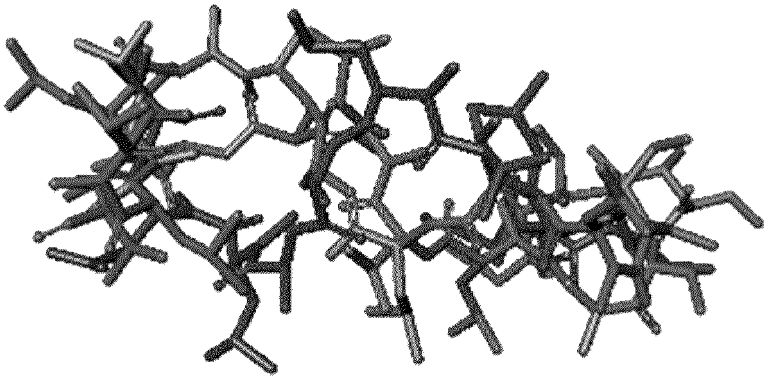

FIG. 2

COMPOUND 1

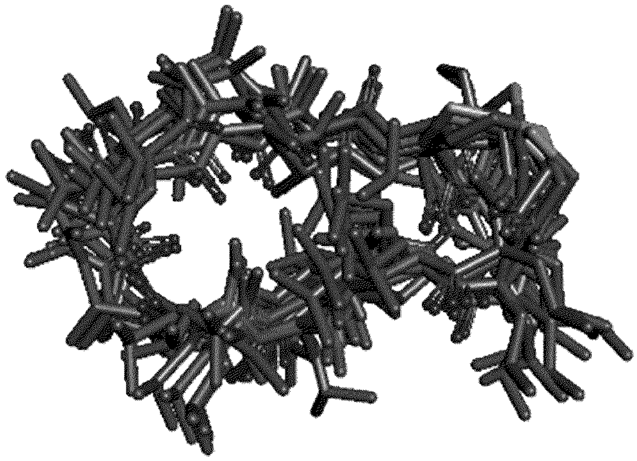

COMPOUND 2

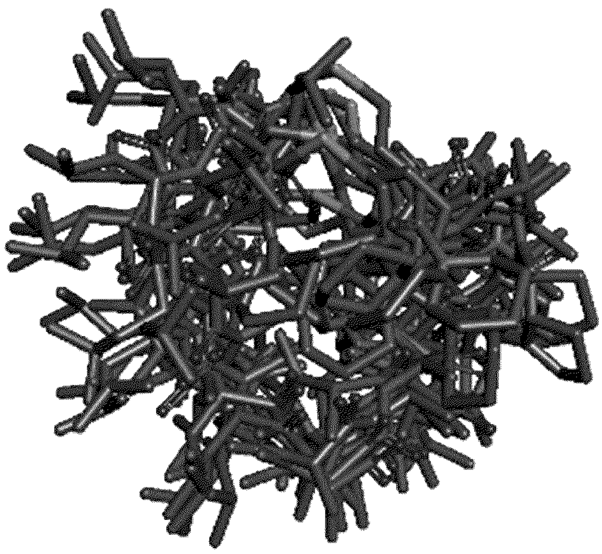

CYCLOSPORIN A

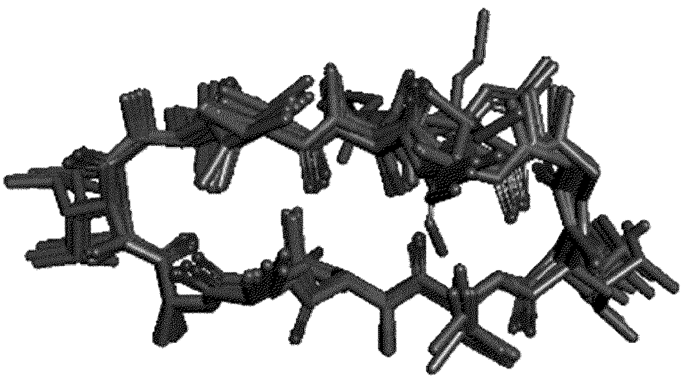

FIG. 3

COMPOUND 1

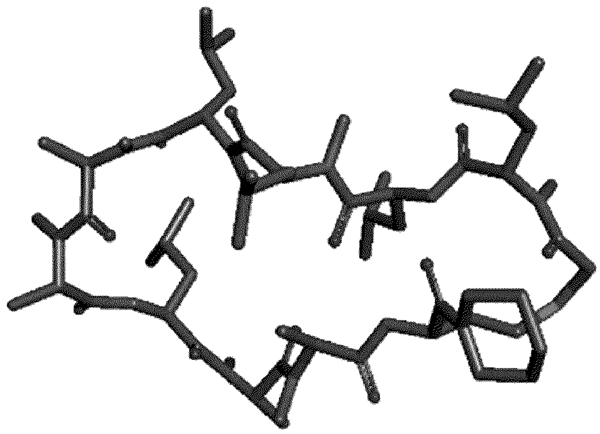

COMPOUND 2

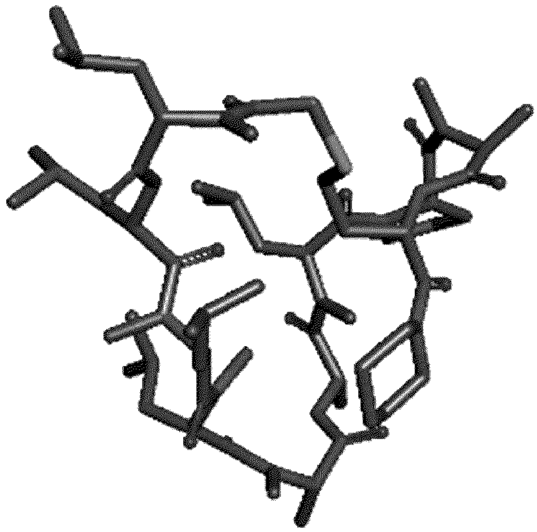

CYCLOSPORIN A

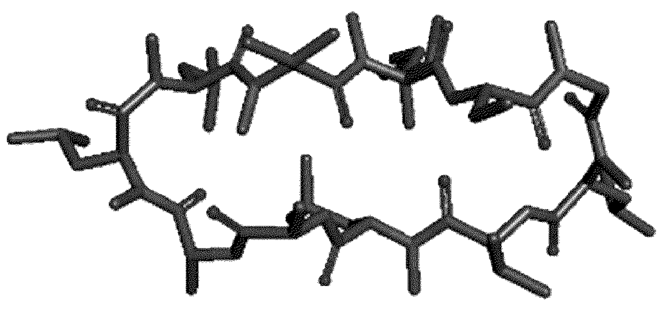

FIG. 4

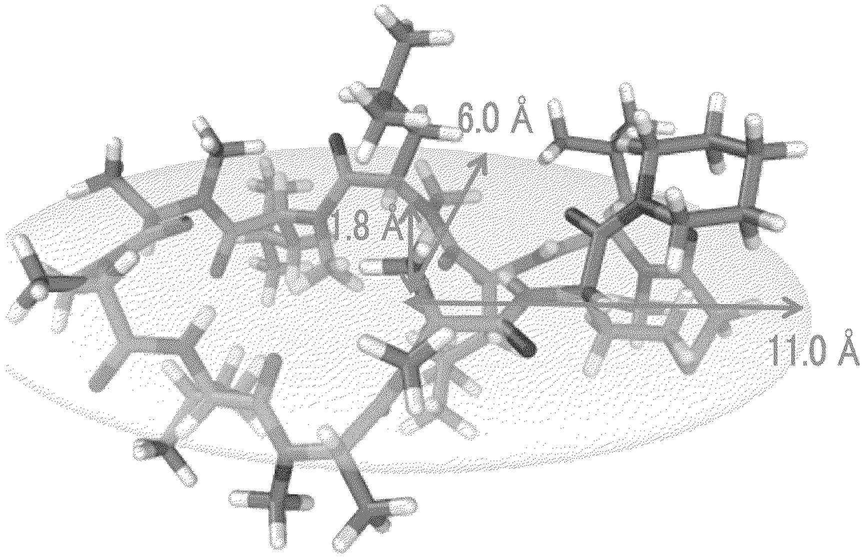


FIG. 5

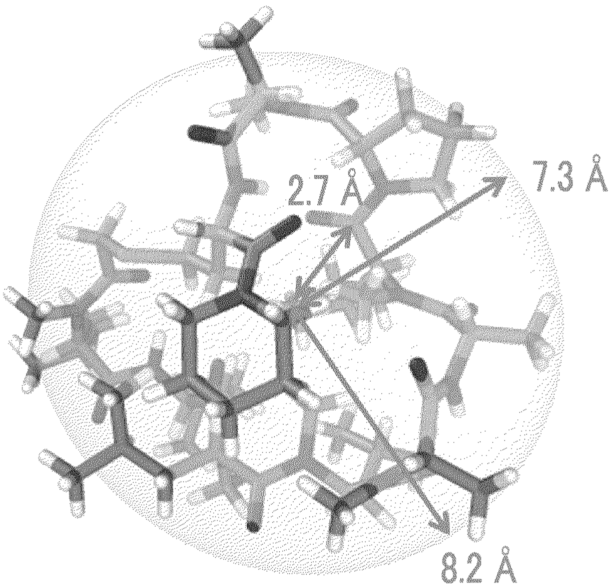


FIG. 6

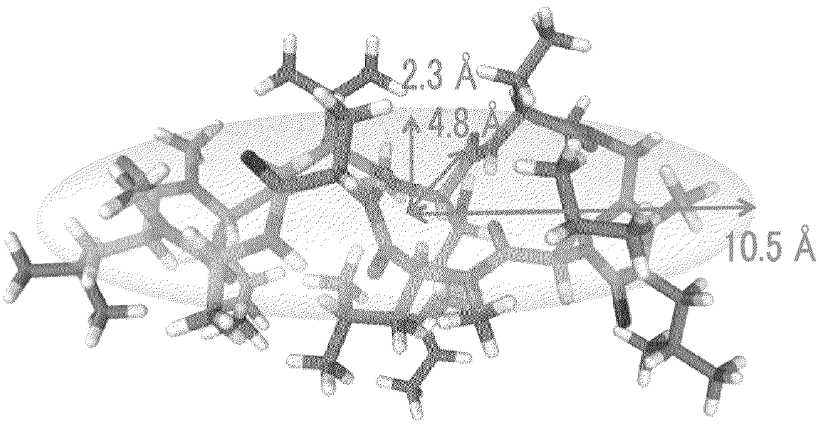

FIG. 7

CYCLOSPORIN A

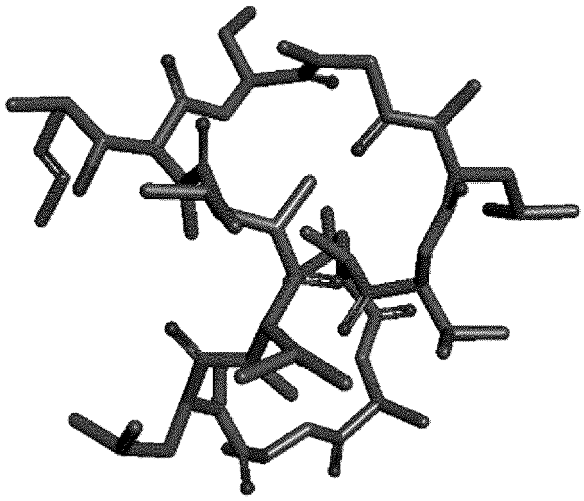

ISOCYCLOSPORIN

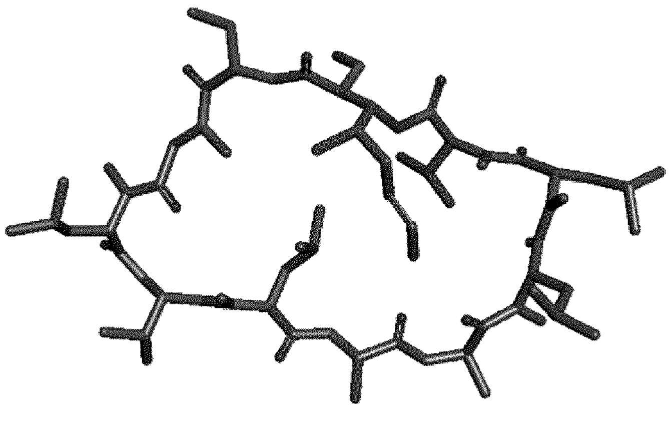

FIG. 8

CYCLOSPORIN A

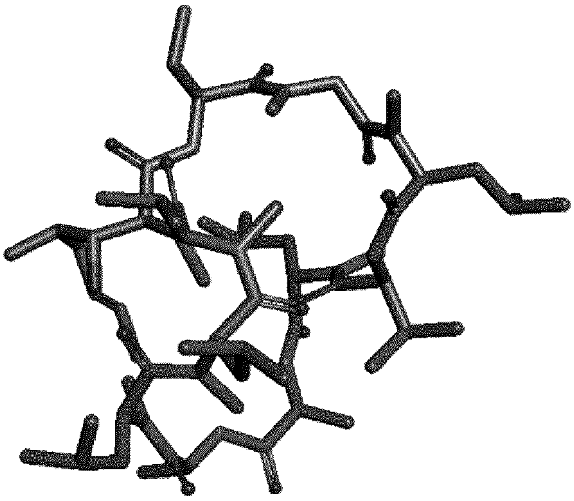

ISOCYCLOSPORIN

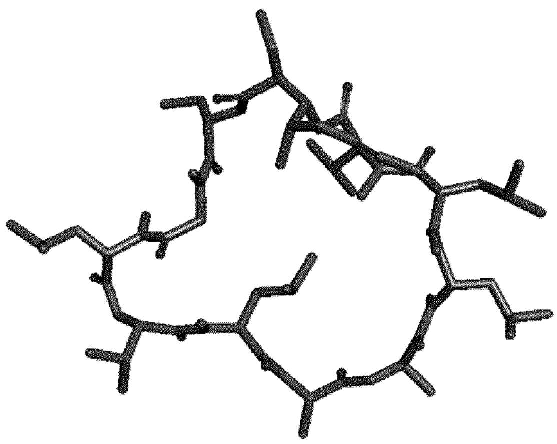

FIG. 9

CYCLOSPORIN A

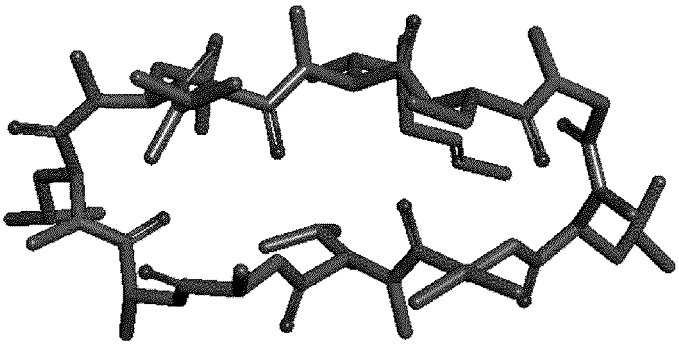

ISOCYCLOSPORIN

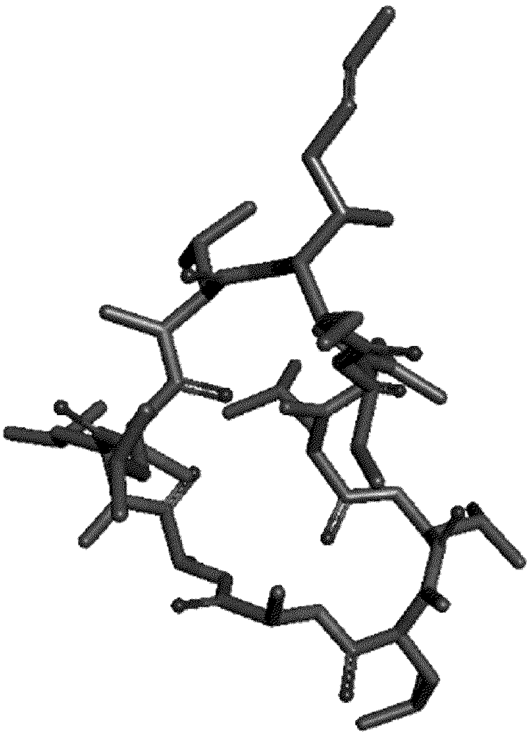

FIG. 10

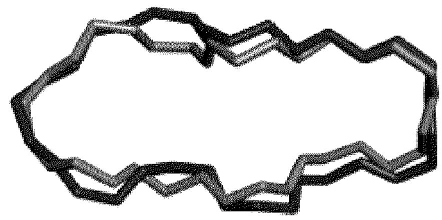

FIG. 11

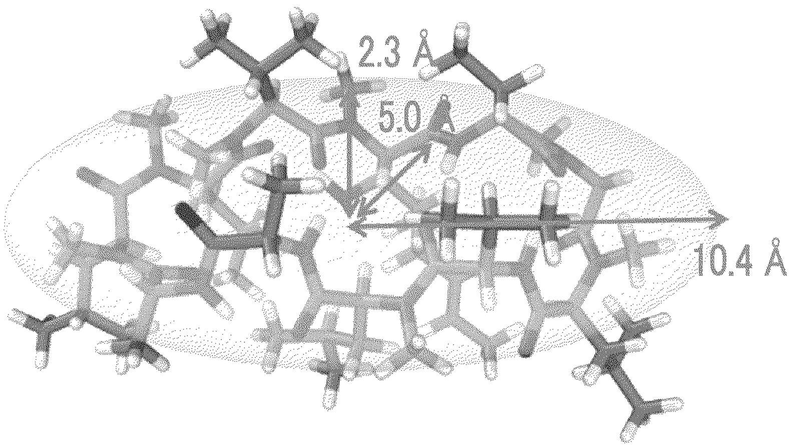


FIG. 12

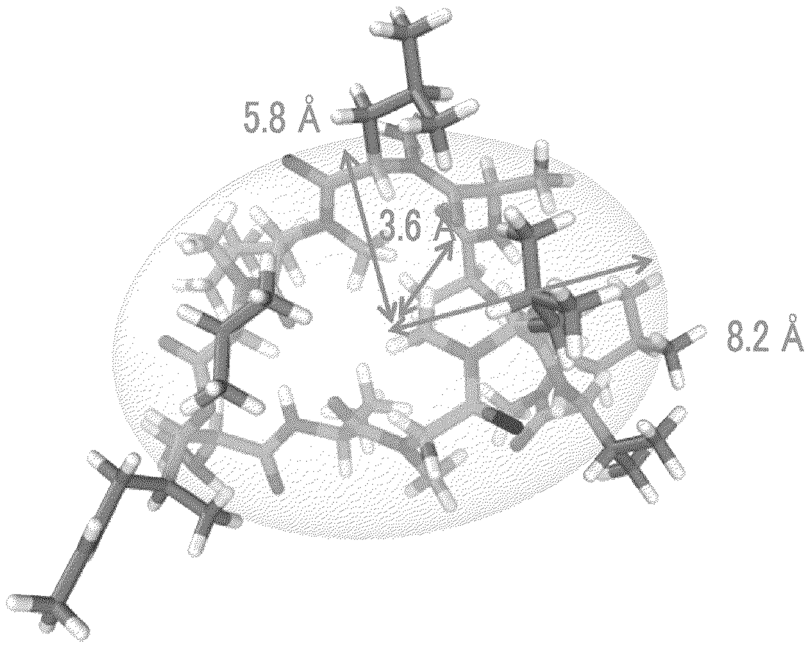

## INTERNATIONAL SEARCH REPORT

International application No.
**PCT/JP2023/030224**

**A. CLASSIFICATION OF SUBJECT MATTER**

***C07K 2/00***(2006.01)i
FI: C07K2/00 ZNA

According to International Patent Classification (IPC) or to both national classification and IPC

**B. FIELDS SEARCHED**

Minimum documentation searched (classification system followed by classification symbols)

C07K2/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | 山田雄太　他, 機械学習を用いた環状ペプチドの膜透過性予測方法の開発, 情報処理学会研究報告, 2019, vol. 2019-BIO-57, no. 13, pp. 1-8<br>outline, (YAMADA, Yuta et al. Developement of membrane permeability prediction method for cyclic peptides with machine learning. IPSJ SIG technical reports.) | 1-10 |
| A | JP 2022-131959 A (TOKYO INST TECH) 07 September 2022 (2022-09-07)<br>claims, fig. 3 | 1-10 |
| A | PARTRIDGE, A. W. et al. Incorporation of Putative Helix-Breaking Amino Acids in the Design of Novel Stapled Peptides: Exploring Biophysical and Cellular Permeability Properties. Molecules. 2019, vol. 24, no. 12, p. 2292, DOI 10.3390/molecules24122292<br>abstract, fig. 1, 3.8, 3.9, fig. 7 | 1-10 |
| A | LAWRENCE, N. et al. Cyclic peptide scaffold with ability to stabilize and deliver a helical cell-impermeable cargo across membranes of cultured cancer cells. RSC Chemical Biology. 2020, vol. 1, pp. 405-420, DOI 10.1039/d0cb00099j<br>abstract, fig. 1, table 1, fig. 3, 4 | 1-10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 October 2023** | **17 October 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

INTERNATIONAL SEARCH REPORT

International application No.
**PCT/JP2023/030224**

**C. DOCUMENTS CONSIDERED TO BE RELEVANT**

| Category* | Citation of document, with indication, where appropriate, of the relevant passages | Relevant to claim No. |
| --- | --- | --- |
| A | WANG, Y. T. et al. Computational modeling of cyclic peptide inhibitor-MDM2/MDMX binding through global docking and Gaussian accelerated molecular dynamics simulations. Journal of Biomolecular Structure and Dynamics. 2021, vol. 39, no. 11, pp. 4005-4014, DOI org/10.1080/07391102.2020.1773317<br>Abstract | 1-10 |
| A | JP 2015-42159 A (UNIV TOKYO) 05 March 2015 (2015-03-05)<br>claims, examples | 1-10 |
| A | WO 2021/141684 A1 (BRISTOL-MYERS SQUIBB COMPANY) 15 July 2021 (2021-07-15)<br>claims | 10 |

Form PCT/ISA/210 (second sheet) (January 2015)

**INTERNATIONAL SEARCH REPORT**
**Information on patent family members**

International application No.
**PCT/JP2023/030224**

| Patent document cited in search report | Publication date (day/month/year) | Patent family member(s) | Publication date (day/month/year) |
|---|---|---|---|
| JP 2022-131959 A | 07 September 2022 | US 2022/0277224 A1<br>claims, fig. 3<br>EP 4102506 A1<br>CN 115050424 A | |
| JP 2015-42159 A | 05 March 2015 | US 2016/0209421 A1<br>claims, examples<br>EP 3040417 A1 | |
| WO 2021/141684 A1 | 15 July 2021 | JP 2023-510737 A<br>claims<br>US 2023/0065827 A1<br>EP 4087857 A1<br>CN 114929728 A<br>KR 10-2022-0122752 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- WO 2018225864 A
- WO 2020122182 A
- WO 2015030014 A
- WO 2013100132 A
- WO 2008117833 A
- WO 2012074129 A

### Non-patent literature cited in the description

- *Nat. Chem.*, 2016, vol. 8, 1105-1111
- *Proc. Natl. Acad. Sci. USA*, 2013, vol. 110, E3445
- *Acc. Chem. Res.*, 2008, vol. 41, 1331-1342
- *Nat. Chemical Biology*, 2011, vol. 7, 810-817
- *ACS Med. Chem. Lett.*, 2014, vol. 5, 1167-1172
- *J. Med. Chem.*, 2018, vol. 61, 4189-4202
- *J. Med. Chem.*, 2018, vol. 61, 11169-11182
- *J. Am. Chem. Soc.*, 2006, vol. 128, 14073-14080
- *J. Chem. Inf. Model.*, 2016, vol. 56, 1547-1562
- *J. Phys. Chem. B*, 2018, vol. 122, 2261-2276